# EUROPEAN PATENT SPECIFICATION

(11) **EP 2 173 867 B1**
(45) Date of publication and mention of the grant of the patent: **25.02.2015**
(21) Application number: 08766775.4
(22) Date of filing: 05.06.2008
(51) Int. Cl.: C12N 9/24, C12N 15/82

(54) **NOVEL POLYGALACTURONASES AND THEIR USES**
NEUE POLYGALACTURONASEN UND DEREN VERWENDUNGEN
NOUVELLES POLYGALACTURONASES ET LEURS UTILISATIONS

(30) Priority: 26.07.2007 EP 07113263; 26.07.2007 US 952046 P
(43) Date of publication of application: 14.04.2010
(73) Proprietor: Monsanto Holland B.V., 1601 BM Enkhuizen (NL)
(72) Inventor: VAN HEUSDEN, Adriaan Willem, NL-6706 KC Wageningen (NL); GORGUET, Benoit, 84000 Avignon (FR)
(74) Representative: Nederlandsch Octrooibureau
(86) International application number: PCT/NL2008/050352
(87) International publication number: WO 2009/014430

(56) References cited:
- WO-A-00/78982
- WO-A-96/30529
- BENOIT GORGUET ET AL: "High-resolution fine mapping of ps-2, a mutated gene conferring functional male sterility in tomato due to non-dehiscent anthers" THEORETICAL AND APPLIED GENETICS ; INTERNATIONAL JOURNAL OF PLANT BREEDING RESEARCH, SPRINGER-VERLAG, BE, vol. 113, no. 8, 25 August 2006 (2006-08-25), pages 1437-1448, XP019457300 ISSN: 1432-2242
- FERRÁNDIZ CRISTINA: "Regulation of fruit dehiscence in Arabidopsis" JOURNAL OF EXPERIMENTAL BOTANY, OXFORD UNIVERSITY PRESS, GB, vol. 53, no. 377, October 2002 (2002-10), pages 2031-2038, XP002421142 ISSN: 0022-0957
- JENKINS E S ET AL: "Dehiscence-related expression of an Arabidopsis thaliana gene encoding a polygalacturonase in transgenic plants of Brassica napus" PLANT CELL AND ENVIRONMENT, XX, XX, vol. 22, no. 2, February 1999 (1999-02), pages 159-167, XP002421141 ISSN: 0140-7791
- 'Instructions for Authors', [Online] Retrieved from the Internet: <URL:http://www.springer.com/life+sciences/ plant+sciences/journal/122>

## Description

### Field of the invention

The present invention relates to the field of plant breeding, in particular the breeding of tomato plants. The invention extents to the fields of both classical and molecular plant genetics and relates to sequences of novel polygalacturonases and their use in marker assisted breeding or in transgenic plants, e.g. to produce plants with a positional sterile phenotype due to non-dehiscent anthers.

### Background of the invention

In higher plants mature pollen are released from the anther by dehiscence, which consists of a succession of cell destructions occurring successively in the tapetum, the septum and ultimately in the stomium. After degeneration of the tapetum, endothecium cells enlarge and lignified fibrous bands are deposited creating a thickening of the cell walls. Subsequently, dehydration and shrinkage of the endothecium and connective cells surrounding the locules create a breaking force in the stomium that eventually leads to pollen release (Keijzer 1987).

Recent progress was made in understanding the molecular control of anther dehiscence, which involves mainly the discovery of the implication of Jasmonic Acid (JA) and ethylene. Several mutants affecting diverse steps in the synthesis of JA in the anthers were identified in Arabidopsis. The observed phenotypes resulted in delay of anther dehiscence (reviewed by Scott et al, 2004). The role of ethylene signalling in this phenomenon was highlighted by Rieu et al. (2003) who observed a delay in the dehiscence of anthers of ethylene insensitive tobacco plants.

Polygalacturonases (PGs) belong to one of the largest hydrolase families (Torki et al, 2000; Markovic and Janecek, 2001). PGs activities have been shown to be associated with a wide range of plant developmental programs (reviewed by Hadfield and Bennett 1998), among them, anther dehiscence: Activity of PGs has been observed in the dehiscence zone of anthers of maize, tobacco, oilseed rape and Arabidopsis (Dubald et al 1993; Sander et al 2001). However their role in the dehiscence process has never been studied into details.

We have recently fine mapped the *positional sterility*-*2* gene, conferring non-dehiscent anthers in tomato (Gorguet et al 2006). It is an object of the present invention to provide for the nucleotide and amino acid sequences of the *ps-2* gene, as well as methods wherein these sequences are used in marker assisted breeding or in transgenic plants, e.g. to produce plants with a positional sterile phenotype due to non-dehiscent anthers and/or to alter fruit ripening.

### Description of the invention

### Definitions

In this description, unless indicated otherwise, the terms and definitions used herein are those used in (Mendelian) genetics, for which reference is made to M.W. Strickberger, Genetics, second Edition (1976), in particular pages 113-122 and 164-177. As mentioned therein, "gene" generally means an inherited factor that determines a biological characteristic of an organism (i.c. a tomato plant), an "allele" is an individual gene in the gene pair present in the (diploid) tomato plant. In this context it is understood that the term a ps-2-gene or -allele as used herein refer to an allele of the a Dehiscence Polygalacturonase (DPG), that is capable of producing or contributing to the positional sterility phenotype of the invention. A *ps*-2-gene or -allele as used herein may thus refer to any loss of (DPG-)function allele that is capable of producing or contributing to the phenotypes of the invention of positional sterility and/or non-dehiscent anthers. A preferred example of a DPG gene is the Tomato Dehiscence Polygalacturonase (TDPG) gene described herein. A preferred example of a *ps*-2-gene or -allele is the particular tomato *ps-2* allele described herein that has a C as last nucleotide of the 3' end of the fifth exon of the TDPG gene.

A polygalacturonase (EC 3.2.1.15) is herein understood as an enzyme that katalyses the random hydrolysis of 1,4-alpha-D-galactosiduronic linkages in pectate and other galacturonans. Polygalacturonases are also referred to as pectin depolymerases or pectinases. Polygalacturonase activity is determined quantitatively by means of colorimetric detection of reducing sugar release as described previously (Parenicova et al., 1998, Eur. J. Biochem. 251:72-80), whereby 1 unit of polygalacturonase activity is defined as the amount of enzyme capable of releasing 1 µmole of reducing sugar ends per minute from polygalacturonic acid (Sigma) as the model substrate at 30°C in 50 mM sodium acetate buffer, pH 4.2, with 0.25 % (w/v) substrate concentration.

A plant is called "homozygous" for a gene when it contains the same alleles of said gene, and "heterozygous" for a gene when it contains two different alleles of said gene. The use of capital letters indicates a dominant (form of a) gene and the use of small letters denotes a recessive gene: "X,X" therefore denotes a homozygote dominant genotype for gene or property X; "X,x" and "x,X" denote heterozygote genotypes; and "x,x" denotes a homozygote recessive genotype. As commonly known, only the homozygote recessive genotype will generally provide the corresponding recessive phenotype (i.e. lead to a plant that shows the property or trait "x") whereas the heterozygotic and homozygote dominant genotypes will generally provide the corresponding dominant phenotype (i.e. lead to a plant that shows the property or trait "X"), unless other genes and/or factors such as multiple alleles, suppressors, codominance etc. (also) play a role in determining the phenotype.

As a general rule, hybrid seed is obtained by crossing two different parent tomato plants, which most often belong to different lines. Using cultivation techniques and plant breeding techniques known per se, such hybrids can be provided with highly specific, desired properties, which makes it possible to "design" the hybrids, i.e. to confer to the hybrid plants predetermined inheritable characteristics. This is usually achieved by suitably choosing (the properties of) the two parent lines which are crossed to provide the hybrid seed. These are usually inbred lines, obtained by self-fertilization (self-pollination) over several generations, and such inbred lines will usually again have been specifically "designed" by the breeder so as to provide hybrid offspring with the desired properties, when crossed with another - usually predetermined - inbred parent line. As a rule, such parent lines will be genetically homozygote and identical (i.e. as a result of inbreeding) so that they can provide, in a stable and reliable manner, genetically uniform -albeit heterozygote- hybrid line combinations, which can combine the properties of the parent lines. In doing so, the aim is on the one hand to cross certain properties from the parent lines as purely as possible into the seed, while on the other hand use is made of the known effect of heterosis or inbred growth, which can provide improved properties regarding -inter alia- the growth of plants and fruits and thereby of the yield. This heterosis effect is obtained when/because the parent lines used are not related with respect to certain genetic properties (i.e. when the parent lines genetically "lie far apart"). For a further description of plant breeding techniques in general, and tomatoes in particular, using classical cultivation techniques, including the formation of hybrids, reference is made to the known handbooks.

As used herein, the term "plant" includes the whole plant or any parts or derivatives thereof, such as plant cells, plant protoplasts, plant cell tissue cultures from which tomato plants can be regenerated, plant calli, plant cell clumps, and plant cells that are intact in plants, or parts of plants, such as embryos, pollen, ovules, fruit (e.g. harvested tomatoes), flowers, leaves, seeds, roots, root tips and the like.

Botanical terminology: Linnaeus is considered the father of botanical classification. Although he first categorized the modem tomato as a *Solanum,* its scientific name for many years has been *Lycopersicon esculentum.* Similarly, the wild relatives of the modem tomato have been classified within the *Lycopersicon* genus, like *L. pennellii*, *L. hirsutum, L. peruvianum, L. chilense*, *L. parviflorum, L. chmielewskii, L. cheesmanii, L. cerasiforme, and L. pimpinellijolium.* Over the past few years, there has been debate among tomato researchers and botanists whether to reclassify the names of these species. The newly proposed scientific name for the modem tomato is *Solanum lycopersicum.* Similarly, the names of the wild species may be altered. *L. pennellii* may become *Solanum pennellii, L. hirsutum* may become *S. habrochaites, L. peruvianum* may be split into *S. 'N peruvianumr'* and *S. 'Callejon de Huayles', S. peruvianum,* and *S. corneliomuelleri, L. parviflorum* may become *S. neorickii, L. chmeilewskii* may become *S. chmielewskii*, *L. chilense* may become *S. chilense*, *L. cheesmaniae* may become S. *cheesmaniae* or *S. galapagense,* and *L.pimpinellifolium* may become S. *pimpinellifolium* (Solanacea Genome Network (2005) Spooner and Knapp; http://www.sgn.cornell.edu/help/about/solanum_nomenclature.html).

Nucleic acid sequences or fragments comprising *ps-2* or DPG genes and alleles may also be defined by their capability to "hybridise" with SEQ ID NO: 1, preferably under moderate, or more preferably under stringent hybridisation conditions. Stringent hybridisation conditions are herein defined as conditions that allow a nucleic acid sequence of at least about 25, preferably about 50 nucleotides, 75 or 100 and most preferably of about 200 or more nucleotides, to hybridise at a temperature of about 65°C in a solution comprising about 1 M salt, preferably 6 x SSC or any other solution having a comparable ionic strength, and washing at 65°C in a solution comprising about 0,1 M salt, or less, preferably 0,2 x SSC or any other solution having a comparable ionic strength. Preferably, the hybridisation is performed overnight, i.e. at least for 10 hours and preferably washing is performed for at least one hour with at least two changes of the washing solution. These conditions will usually allow the specific hybridisation of sequences having about 90% or more sequence identity. Moderate conditions are herein defined as conditions that allow a nucleic acid sequences of at least 50 nucleotides, preferably of about 200 or more nucleotides, to hybridise at a temperature of about 45°C in a solution comprising about 1 M salt, preferably 6 x SSC or any other solution having a comparable ionic strength, and washing at room temperature in a solution comprising about 1 M salt, preferably 6 x SSC or any other solution having a comparable ionic strength. Preferably, the hybridisation is performed overnight, i.e. at least for 10 hours, and preferably washing is performed for at least one hour with at least two changes of the washing solution. These conditions will usually allow the specific hybridisation of sequences having up to 50% sequence identity. The person skilled in the art will be able to modify these hybridisation conditions in order to specifically identify sequences varying in identity between 50% and 90%.

### Detailed description of the invention

We have recently fine mapped the *positional sterility-2* gene, conferring non-dehiscent anthers in tomato (Gorguet et al 2006). We have now isolated the *ps-2* gene by positional cloning. Subsequent characterisation of the *ps-2* gene revealed that the wild type gene codes for a novel polygalacturonase (PG) and that a single mutation in the coding sequence of gene is responsible for this phenotype of positional sterility due to non-dehiscent anthers. We found that this mutation affects one of the intron splicing recognition site of the gene giving rise to an aberrant mRNA, lacking one of the exons. We have further found that this new PG gene, hereafter designated as Dehiscence PG (DPG) is also expressed in maturing fruits. The jasmonic acid and ethylene play a role in the control of expression of DPG and DPG is involved in the process of fruit ripening.

In a first aspect therefore, the present invention relates to an nucleic acid molecule, preferably, an isolated nucleic acid molecule, as defined in claim 1.

The nucleic acid molecule comprises a nucleotide sequence encoding a *ps-2-*allele of the dehiscence polygalacturonase gene as defined in claim 1, wherein the dehiscence polygalacturonase is a polypeptide with polygalacturonase activity. Polygalacturonase activity and this activity may be determined is defined hereinabove. The polypeptide with dehiscence polygalacturonase activity comprises an amino acid sequence that has 90, 95, 98, 99 or 100% sequence identity with the amino acid sequence of SEQ ID NO. 2.

A preferred nucleotide sequence of the invention is from a species within the genus *Solanum.* More preferably, the nucleotide sequence is from a species within the *Solanum Lycopersicum* complex, including e.g. *S. lycoperisicum*, *S. chmielewskii, S. habrochaites, S. pimpinellifolium, S. neorickii,* and *S. pennellii.* Alternatively, the nucleotide sequence may be from *Solanum melongena.*

Alternatively, the nucleic acid molecule of the invention is a molecule that comprises a nucleotide sequence of at least 10, 12, 14, 16, 18, 20, 22, 24, 26, 28, 30, 35, 40 or 50 contiguous nucleotides from SEQ ID NO: 1 as defined in claim 1. It is understood that the nucleic acid molecule may comprises more than one stretch of such contiguous nucleotides, which may be directly linked together or separated by other sequences.

In a second aspect the invention relates to a method for detecting, isolating, amplifying and/or analysing a DPG allele in a plant, preferably in a plant of the genus *Solanum,* more preferably in a plant of the *Solanum Lycopersicum* complex, or a *Solanum melongena* plant. The method comprises at least the step of providing a sample comprising nucleic acids of the plant and hybridising the nucleic acids of the plant with a nucleic acid molecule comprising a nucleotide sequence of at least 10 contiguous nucleotides from a nucleotide sequence as defined in claim 1. Preferably the nucleic acid molecule for hybridisation with the sampled nucleic acids of the plant comprises a nucleotide sequence of at least 12, 14, 16, 18, 20, 22, 24, 26, 28, 30, 35, 40 or 50 contiguous nucleotides from a nucleotide sequence as defined above. It is understood that in case the nucleic acid molecule for hybridisation is a single stranded molecule it may also comprise the complement, i.e. opposite strand, of the nucleotide sequence as defined above.

Method for detection, isolation, amplification and/or analysis of specific nucleic acid sequences, such as a DPG allele in a plant, preferably in a plant of the genus *Solanum,* rely on the sequence specific hybridisation of a nucleic molecule comprising a predetermined sequence, i.e. the nucleic acid molecule for hybridisation, to nucleic acids in the sample to be detected, isolated, amplified and/or analysed. In such method the nucleic acid molecule for hybridisation may thus any nucleic acid molecule capable of hybridising to a DPG allele as defined in claim 1, preferably a *Solanum* DPG allele. The molecule may be a probe, e.g. a hybridisation probe or may be a primer to be extended by a polymerase in extension or amplification reaction.

Generally methods for detection, isolation, amplification and/or analysis of specific nucleotide sequences that rely on sequence specific hybridisation of a nucleic molecule comprising a predetermined sequence are well known in the art (see e.g. Sambrook and Russell, 2001, "Molecular Cloning: A Laboratory Manual", 3rd edition, Cold Spring Harbor Laboratory, Cold Spring Harbor Laboratory Press, New York). More specifically, methods for detection, isolation, amplification and/or analysis of specific DPG allele sequences of the invention may include PCR Amplification (U.S. Pat. Nos. 4,683,195; and 4,683,202; PCR Technology: Principles and Applications for DNA Amplification, ed. H. A. Erlich, Freeman Press, NY, N.Y., 1992), Allele-specific PCR (Gibbs, 1989, Nucleic Acid Res. 17:12427-2448), Allele Specific Oligonucleotide Screening Methods (ASO; Salki et al., 1986, Nature 324:163-166), ligase-mediated allele detection methods such as the Oligonucleotide Ligation Assays (OLA; 1988, Landegren et al., Science 241:107-1080) or the ligation amplification reaction (Wu et al., 1989, Genomics 4:560-569; Barany, 1990, Proc. Nat. Acad. Sci. 88:189-193), Denaturing Gradient Gel Electrophoresis (in Chapter 7 of Erlich, ed., 1992, PCR Technology, Principles and Applications for DNA Amplification, W. H. Freeman and Co., New York), Temperature Gradient Gel Electrophoresis (TGGE), Single-Strand Conformation Polymorphism Analysis (Orita et al., 1989, Proc. Nat. Acad. Sci. 85:2766-2770), differential chemical cleavage of mismatched base pairs (Grompe et al., 1991, Am. J Hum. Genet. 48:212-222), enzymatic cleavage of mismatched base pairs (Nelson et al., 1993, Nature Genetics 4:11-18), systems such as TaqMan™ (Perkin Elmer), Invader Assay, which includes isothermic amplification that relies on a catalytic release of fluorescence (Third Wave Technology at www.twt.com), massive parallel sequencing (e.g. of Roche / 454 Life Sciences performed on a GS 20 Genome Sequencer) to produce sequence information for a given locus in thousands of individuals, optionally combined with a 3D pooling strategy (see e.g the Keypoint™ technology at www.keygene.com).and the like.

A method of the invention is a method for detection, isolation, amplification and/or analysis of a *ps*-2-allele, whereby the *ps*-2-allele is an allele that has a C, A or T as last nucleotide of the 3' end of the fifth exon of the DPG gene, preferably the allele has a C as last nucleotide of the 3' end of the fifth exon of the DPG gene. This position corresponds to the G at position 3772 of SEQ ID NO:1. Alternatively, molecular markers specific for DPG alleles may be developed. A "molecular marker" is herein understood to refer to a nucleic acid sequence, or a set thereof, that is indicative (directly or indirectly) for the presence or absence of a particular allele, e.g. a DPG allele or a *ps-2* allele as herein defined. The presence or absence of the molecular marker can be detected in a wide variety of molecular assays or tests including e.g. Restriction Fragment Length Polymorphisms (RFLPs), Randomly Amplified Polymorphic DNAs (RAPDs), Arbitrarily Primed Polymerase Chain Reaction (AP-PCR), DNA Amplification Fingerprinting (DAF), Sequence Characterized Amplified Regions (SCARs), and Amplified Fragment Length Polymorphisms (AFLPs). A molecular marker specific for a DPG allele is herein understood as a marker that is present within the genome of the plant, preferably the *Solanum* plant no more than 100, 50, 20, 10, 5 or 2 kb from the a nucleotide sequence encoding the polypeptide with polygalacturonase activity as defined hereinabove or a part thereof. Such molecular markers may be used for detection of specific DPG alleles, including *ps*-2-alleles.

In a third aspect the invention relates to the use of a nucleic acid molecule for hybridisation as defined in herein above in marker-assisted breeding. The marker-assisted breeding comprises the detection of a *ps*-2-allele. A *ps*-2-allele is herein understood as a DPG allele of a plant, preferably a plant of the genus *Solanum,* more preferably in a plant of the *Solanum Lycopersicum* complex or in a *Solanum melongena* plant, that, when homozygous produces a positional sterility phenotype that is due to non-dehiscent anthers. A *ps*-2-allele will usually be an allele from which in a plant that is homozygous for the allele insufficient DPG activity is expressed to prevent positional sterility and non-dehiscent anthers. The marker-assisted breeding comprises the detection of a *ps*-2-allele that has a C, A or T as last nucleotide of the 3' end of the fifth exon of the DPG gene, of which a C as last nucleotide of the 3' end of the fifth exon of the DPG gene is most preferred.

Also described herein is a method for producing a plant with non-dehiscent anthers. Preferably the plant is a *Solanum* plant, more preferably a plant of the *Solanum Lycopersicum* complex, or a *Solanum melongena* plant. The plant preferably has a phenotype of positional sterility. The method comprises the steps of: (a) crossing a first plant with a second plant that is homozygous for a *ps*-2-allele; (b) backcrossing the F1 generation and further generations for at least one (preferably at least two) generation with the first plant as recurrent parent; and, (c) selfing the furthest backcrossed generation obtained in b) for at least one (preferably at least two) generations; wherein a molecular marker is used in at least one of steps b) and c) to select for a plant that is homozygous for the *ps*-2-allele. The molecular marker is a marker specific for a *ps-2-*allele as herein defined and. is or detects a C, A or T as last nucleotide of the 3' end of the fifth exon of the DPG gene, of which a C as last nucleotide of the 3' end of the fifth exon of the DPG gene is preferred.

In a fifth aspect the invention relates to methods for producing a plant with a mutation or genetic modification in a DPG-allele as defined in claim 1. Preferably the plant is a *Solanum* plant, more preferably a plant of the *Solanum Lycopersicum* complex, or a *Solanum melongena* plant. The mutation or genetic modification may be introduced in the locus of a DPG encoding nucleotide sequence/gene using the technique of TILLING (Targeted Induced Local Lesions IN Genomes). Methods for TILLING are well known in the art (McCallum et al., 2000 Nat Biotechnol. 18(4):455-7; reviewed by Stemple, 2004, Nat Rev Genet. 5(2):145-50.). The TILLING mutagenesis technology is useful to generate and/or identify, and to eventually isolate mutagenised variants of a DPG genes, including *ps*-2-alleles. TILLING also allows selection of plants carrying such mutant variants. TILLING combines high-density mutagenesis with high-throughput screening methods. The steps typically followed in TILLING are: (a) mutagenesis, e.g. by Ethyl methanesulfonate (EMS) or N-ethyl-N-nitrosourea (ENU); (b) growth of plant from mutagenised seed, and optionally, backcrossing the plants for at least one generation; (c) preparation of DNA from tissue samples of the plants and, optionally, pooling of DNAs from individuals; (d) PCR amplification of a region of interest, i.e. the DPG gene, e.g using primers as hereinabove defined; (e) detection of mutant PCR products (f) identification of the mutant individual and (h) optionally, sequencing of the mutant PCR. Initially in the method detection of mutant PCR products in step (e) and optionally (f) was performed by denaturation and annealing to allow formation of heteroduplexes and subsequent detection of heteroduplexes by DHPLC (Denaturing High Perfomance Liquid Chromatography) (McCallum et al., 2000, *supra*). The method was made more high throughput by using the restriction enzyme Cel-I combined with a gel based system to identify mutations (Colbert et al., 2001, Plant Physiol. 126(2):480-4). Other methods for detection or identification of single base mutations in the target DPG gene that may be used in methods of TILLING include e.g. resequencing DNA as has been described (see e.g. Slade et al., 2005, Nat Biotechnol. 23(1):75-81) or massive parallel sequencing (e.g. of Roche / 454 Life Sciences performed on a GS 20 Genome Sequencer) to produce sequence information for a given locus in thousands of individuals, optionally combined with a 3D pooling strategy (see e.g the Keypoint™ technology at www.keygene.com). Mutagenesis may be performed by radiation or with a chemical mutagen such as EMS (Lightner and Caspar, 1998, "Seed mutagenesis of Arabidopsis", In: Methods in Molecular Biology: Arabidopsis Protocols (eds. J. M. Martinez-Zapater and J. Salinas), pp. 91-103. Totowa, New Jersey: Humana Press) or ENU (Draper et al., 2004, Methods Cell Biol. 2004;77:91-112). A preferred method for producing a plant with a mutation in a DPG-allele as defined in claim 1 at least includes the steps of: (a) mutagenising seeds of a plant; (b) growing plants of the mutagenised seeds obtained in a); (c) optionally, backcrossing the plants obtained in b) for at least one generation; and (d) screening plants obtained in b) or c) for the presence of a mutation in a DPG-allele. Preferably the plant is a *Solanum* plant, more preferably a plant of the *Solanum Lycopersicum* complex, or a *Solanum melongena* plant. The mutation in the DPG-allele causes the allele to be a *ps*-2-allele that has a C, A or T as last nucleotide of the 3' end of the fifth exon of the DPG gene, preferably the allele has a C as last nucleotide of the 3' end of the fifth exon of the DPG gene.

In a sixth aspect the invention relates to methods for producing a transgenic plant, preferably a *Solanum* plant with non-dehiscent anthers and/or altered fruit ripening.. The method comprises at least the step of transforming a plant cell with a nucleic acid construct as defined in claim 11, wherein presence of the nucleic acid construct in cell of the plant reduces expression of DPG activity to a level that effects positional sterility and non-dehiscent anthers and/or altered fruit ripening. Plants with non-dehiscent anthers may derived from transformed plant cells or from plants comprising such transformed cells by methods known to the skilled person per se.

Thus in a preferred embodiment the nucleotide sequence in the nucleic acid construct is operably linked to a promoter for expression in a plant cell, e.g. a *Solanum* cell, and expression of the nucleotide sequence reduces expression of DPG activity by RNA interference. Methods and nucleic acid constructs for gene silencing are described in US20070130653, US20070074311 and references cited therein. The invention thus relates to a nucleic acid molecule that is a construct comprising at least a fragment of a nucleotide sequence encoding a DPG as defined hereinabove being operably linked to a promoter for expression in a plant cell. In the nucleic acid construct the fragment preferably comprises a sequence of 30, 60, 100, 200 or 500 contiguous nucleotides from a nucleotide sequence having at least 90, 95 or 100% sequence identity to a nucleotide sequence encoding a DPG as defined hereinabove, or a complement thereof, more preferably the nucleotide sequence encoding a TDPG is SEQ ID NO:1.

In another preferred embodiment, the transgenic plants with non-dehiscent anthers and/or altered fruit ripening may be obtained by knock-out of the DPG gene by homologous recombination. In this method the nucleic acid construct is a construct for homologous recombination and the nucleotide sequence encodes a *ps*-2-allele as defined in claim 11, so that it reduces expression of DPG activity to a level that effects positional sterility and non-dehiscent anthers. and/or altered fruit ripening. Homologous recombination allows introduction in a genome of a selected nucleic acid at a defined selected position. Methods for performing homologous recombination in plants have been described not only for model plants (Offringa et al., 1990 EMBO J. 9(10):3077-84) but also for crop plants, for example rice (Iida and Terada, Curr Opin Biotechnol. 2004 April; 15(2):132-8). The nucleic acid to be targeted is thus an deficient DPG allele, e.g. a *ps*-2-allele, used to replace the endogenous DPG gene.

Plants of the invention with the non-dehiscent anthers and/or positional sterile phenotypes have the advantage that they allow more cost-effective production of hybrid plants by avoiding or reducing self-fertilisation. Production of hybrids may involve the use of male sterilities such as CMS, genetic sterility or positional sterility. Positional sterility is preferred because one the one hand it reduces or prevents undesired self-fertilisation whereas on the other hand if so required it does allow self-fertilisation by (manual) opening or damaging of the anthers. The DPG alleles of the invention may thus be used as a tool to prevent inbreeding during the creation of hybrids that may or may not have a positional sterile phenotype due to non-dehiscent anthers (depending on the phenotype of the male parent). In addition the non-dehiscent anthers and/or positional sterile phenotypes may advantageously be applied in the production of seedless fruit in avoiding or reducing (self)-fertilisation and thereby improving the seedless phenotype.

Although the invention has been exemplified by means of tomato plants, it is understood that the invention includes DPG nucleic acids, ps-2 alleles, and methods for producing (transgenic) plants of all commercially important crops including e.g. Asteraceae (including the food crops lettuce, chicory, globe artichoke, sunflower, yacon, safflower) Cucurbitaceae (commonly known as gourds or cucurbits and includes crops like cucumbers, squashes (including pumpkins), luffas, melons and watermelons), Brassica (including swedes, turnips, kohlrabi, cabbages, brussels sprouts, cauliflower, broccoli, mustard seed and oilseed rape), Leguminous Crops (including dry beans,dry broad beans, dry peas, chickpea, garbanzo, bengal gram dry cowpea, black-eyed pea, blackeye bean, pigeon pea, toor, cajan pea, congo bean, lentil, bambara groundnut, earth pea vetch, common vetch Lupins, soybean, peanut) Chenopodiaceae (spinach). Liliaceae (unions, leek), Apiaceae (carrots), Grain crops (rice, barley, wheat, oats, corn), Solanaceae (including tomato, pepper, eggplant).

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one".

### Description of the figures

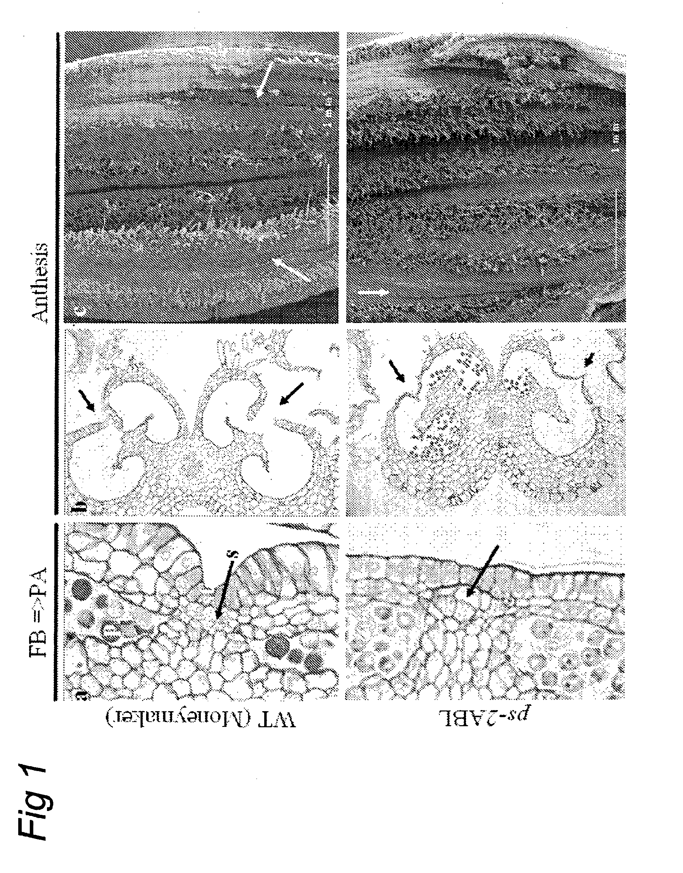

Figure 1: Microscopic observation of anthers of ps-2ABL and Wild-Type (Moneymaker).
   a: Cross section of anthers at late flower bud (FB), early pre-anthesis (PA) stained in toluidine blue. Arrows refer to the crystals that can be seen in the septum cells of WT and ps-2ABL. s: septum; st: stomium; p: pollen.
   b: Cross section of anthers at anthesis stained in toluidine blue. Arrows indicate the opening of the anther in WT and the stomium that remains closed in ps-2ABL.
   c: longitudinal section of anther cones at anthesis observed by SEM. Arrows indicate the longitudinal opening if the anther in WT and the unopened anthers in ps-2ABL.

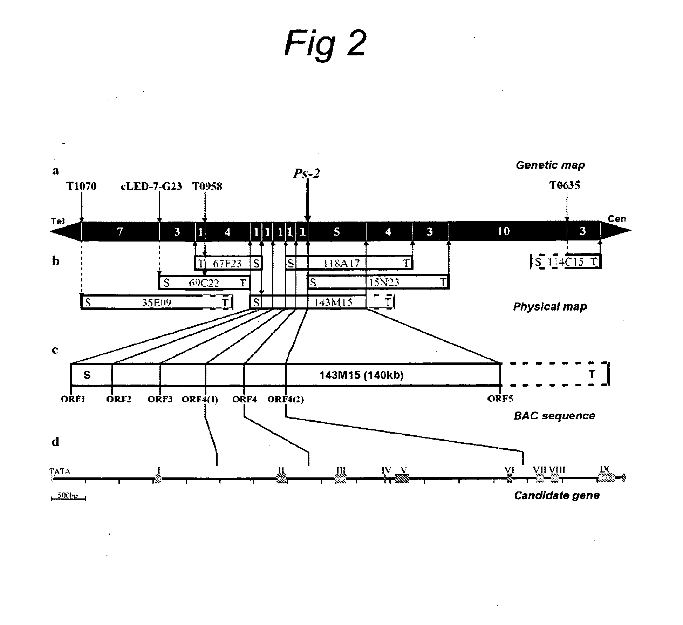

Figure 2: Cloning of the *ps-2* gene, from genetic map to candidate gene
   a: Genetic map developed in a recombinant F2 population (*ps*-2ABL x S. *pimpinellifolium;* Gorguet et al, 2006). In white are the numbers of recombinant plants between each marker.
   b: Physical map: Arrows in dashed represent the computational anchoring of BACs. Arrows in full line represent the anchoring of BACs by molecular markers. (S: SP6; T: T7).
   c: Genetic positions of the ORFs on BAC clone 143M15.
   d: Structure of candidate gene ORF4. Coloured cylinders with roman numbers represent exons. Positions of the putative TATA box and PolyA are indicated by an inverted blue triangle at the beginning of the sequence and a green rhombus at the end of the sequence.
Figure 3: RT-PCR performed on RNA from anther cones at post-anthesis from *ps-*2ABL and *cv*. Moneymaker.
Figure 4: Intron splicing between exon IV and VI of ORF4, in Moneymaker (WT) and inps-2ABL in anthers.
Figure 5: Phylogenetic tree for 20 angiosperm and 1 gymnosperm PG aa sequences. The phylogram is the result of a 50% Majority-rule consensus of 3296 trees (using tree weights). The PG sequences segregate into two major clades identified as the well characterized clades A and B. the candidate protein (TDPG) is part of clade B.
Figure 6: Alignment of Pfam GH28 domain of TFPG, ORF4 and ps-2 (mutant of ORF4). Colour code is only effective for the comparison between TFPG and ORF4: In reverse colour are the identical amino acids. In grey are the conserved substitutions. In bold underlined are the four conserved domains of polygalacturonase as defined by Rao et al (1996).
Figure 7: Tissue specific expression of ORF4. cDNA of Moneymaker from diverse tissues were subjected to PCR amplification using primers dedicated to quantitative PCR analysis. Genomic DNA of Moneymaker was used as control for contamination 1: Leaf abscission zone; 2: Flower abscission zone; 3: Anthers at anthesis; 4 : Fruit at mature stage.
Figure 8: Simplified model for the hormonal control of anther dehiscence and fruit maturation in tomato in which TDPG is taking part.

### Examples

### 1. Example 1

### 1.1 Materials and Methods

### 1.1.1 Plant material

The F₂ recombinant sub-population developed from the cross between *ps*-2ABL (a true Advanced Breeding Line *S. lycopersicum,* homozygous for the *ps-2* mutation) and S. *pimpinellifolium,* was used for genetic mapping. This population, segregating for *ps-2,* is composed of 146 F₂ plants recombinants in the *ps-2* locus region (Gorguet et al, 2006).

Another 176 ABLs, eight of them being *ps-2*/*ps-2,* were used to test the association between the identified SNP and the *ps-2* locus.

Anthers of *ps*-2ABL and cv. Moneymaker were used for microscopic observations.

### 1.1.2 Microscopy

Plant material was fixed for. 24 h at 4°C in 0.1 M phosphate buffer, pH 7.0, containing 4% paraformaldehyde. Samples for scanning electron microscopy were processed as described in Dornelas et al. (2000), and digital images were obtained using an Orion Framegrabber (Matrox Electronic Systems, Unterhaching, Germany). Samples for light microscopy were embedded in Technovit 7100 (Hereaus Kulzer, Wehrheim, Germany), stained with toluidine blue, and mounted in Euparal (Chroma-Gesellschaft, Kongen, Germany).

### 1.1.3 BAC library screening and contig construction

We used the tomato *Hind*III BAC library constructed from genomic DNA of the cultivar accession Heinz 1706. The Heinz library is a 15 genomes equivalent with an average insert size of 114.5kb (Budiman et al., 2000). Screening of the BAC library was performed by PCR amplification, first on plate pools and then on individuals. Plasmid DNA of the positive BAC clones was then isolated and used for further analysis.

BAC ends sequences of the positive BAC clones were obtained from the SGN database (Mueller et al. 2005). Conversion of the BAC ends sequences into CAPS or dCAPS markers was performed as described by Gorguet et al. (2006). Details of the PCR markers derived from BAC ends sequences are presented in Table 1.

**Table 1: PCR markers used in the genetic linkage maps**

| Marker name | Use | Primer sequence | Size (bp) | Restriction enzyme |
|---|---|---|---|---|
| **Markers developed from BAC ends sequences** | | | | |
| 67F23-T | CAPS | Fw: CTACTCTTCCGCCATAACTG | 599 | *Hinc*II |
| | | Rv: GATCCAAACGAACAAAAGTCA | | |
| 67F23-S | CAPS | Fw: TCATTCCGTTGCTGAATGAGA | 413 | *Dro*I |
| | | Rv: ATAACTTATATCACTCCCAATCA | | |
| 69C22-T | dCAPS | Fw: TCTTTCGATATTTTTCAGAACTAA | 200 | *Dde*I |
| | | Rv: TGAGATGTTTGCAATAACATTCT | | |
| 143M15-S | CAPS | Fw: CATCGAAGTAACAGAGATATTA | 369 | *Mwo*I |
| | | Rv: CCATAGGGATTATGATGTGTA | | |
| 114C15-T | CAPS | Fw: GCACTGAAGAATGGATAGACTC | 457 | *MnI*I |
| | | Rv: GGAATTGACCAAAAAGGATAGC | | |
| 113A17-T | CAPS | Fw: GGCATGGTGAAGTCCACATT | 739 | *Hae*III |
| | | Rv: GTGTCACAGGTTTGGTTCAT | | |
| 15N23-T | CAPS | Fw: GGCAGATATCTGCAATACGT | 576 | *Taq*I |
| | | Rv: ATCATGAACAGCAAAACAACCA | | |

| **Markers developed from BAC 143M15 sequence** | | | | |
|---|---|---|---|---|
| ORF1 | CAPS | Fw: CTGTATCTATGACGAGGAGA | 625 | *Hae*III |
| | | Rv: GATCCTGAAGCTGAAGCTT | | |
| ORF2 | CAPS | Fw: AATATTTTCAACTTTCAAATCTCTT | 206 | *Mnl*I |
| | | Rv: ACGAAGGCATGATTGTCGTTA | | |
| ORF3 | CAPS | Fw: GTTGAACTTATACCACTAGGA | 937 | *Nde*I |
| | | Rv: GTGCGGTCTCATCAACTCAA | | |
| ORF4 | dCAPS | Fw: GAACACTTAGGTTAAAATATAGC | 217 | *Alu*I |
| | | Rv: CCTACTATCCTTCTTGTAATCT | | |
| ORF5 | CAPS | Fw: CTTAAAGGCACACTTAGATTCA | 962 | *HpyCH4*IV |
| | | Rv: CTGAGAATTCTCTTGACTGCA | | |
| ORF4(1) | SCAR | Fw: GCTTTATTCATAGTAAATTCTGT | 805/885 | |
| | | Rv: TCAGACAAATCATCGTATATTGA | | |
| ORF4(2) | SCAR | Fw: TCCATTTGTAGTTTCATAAAGC | 465/515 | |
| | | Rv: CCAAGCGGATAATTAATGTCA | | |

| **Marker developed for *ps*-2ABL allele identification within *S***.***lycopersicum*** | | | | |
|---|---|---|---|---|
| *ps-2* marker | CAPS | Fw: CAAATTGGATGAGAGTTTTGAA | 695 | *HpyCH4*IV |
| | | Rv: CATTTTACAAGTGTAACAACTTG | | |

Fingerprinting patterns of individual BAC clones were generated essentially as described by Brugmans et al (2006), using the *Hind* III/*Taq*I enzyme combination.

### 1.1.4 BAC DNA sequencing and analysis

The size of BAC clone 143M15 was estimated by pulse field gel electrophoresis. BAC clone 143M15 was sequenced via the shotgun-sequencing method by Greenomics (The Netherlands). PCR markers derived from the candidate genes identified on the BAC sequence were developed as described by Gorguet et al. (2006). To identify putative genes, the final BAC DNA sequence was scanned against the tomato Unigene database from SGN (Mueller et al, 2005) and the Arabidopsis gene models database from TAIR (http://www.Arabidopsis.org; Huala et al., 2001), using the TBLASTX interface of SGN (Mueller et al., 2005), with a significance threshold of 1E-10. PCR markers based on putative ORF sequences were developed and screened on the recombinant population as described by Gorguet et al. (2006). Details of these PCR markers are given in Table 1.

### 1.1.5 Candidate gene analysis and phylogenetic analysis

The complete genomic DNA sequence of ORF4, as well as the up-stream and down-stream sequences containing respectively the promoter and the gene terminator was amplified and sequenced in Moneymaker and the *ps*-2ABL using several successive overlapping primer pairs giving products of around 900bp. The resulting DNA sequences of Moneymaker and *ps*-2ABL were assembled with DNAStar. Softberry gene finding software was used to identify the putative exons and introns of the candidate gene.

Tomato polygalacturonase protein sequences with known tissue expression, as well as the best hits of a protein BLAST search with the candidate protein, were selected to conduct a phylogenetic analysis. Only the Pfam Glycosyl Hydrolase 28 domains, of the selected protein sequences, were used for the analysis. The Pfam GH28 domain of each protein sequence was identified with the protein Blast interface of NCBI. Selected amino acid sequences of Pfam GH28 domains were aligned using ClustalW multiple sequence alignment software (Higgins et al, 1994).

PAUP software package version 4 (Swofford 2002) was used to construct a 50% majority-rule consensus phylogenetic tree using maximum parsimony (1000 bootstrap replicates and 250 addition sequences replicates). Cedar PG protein sequence was used and defined as outgroup.

The amino acid sequences and their protein identification numbers were: Kiwi fruit (AAC14453; Atkinson and Gardner, 1993), grape berry fruit (AAK81876; Nunan et al, 2001), soybean pods (AAL30418; Christiansen et al, 2002), peach fruit (CAA54448; Lester et al, 1994), apple fruit (AAA74452; Atkinson, 1994), pear fruit (BAC22688; Hiwasa et al, 2003), Arabidopsis dehiscence zone ADPG1 (CAA05525; Sander et al, 2001), oilseed rape dehiscence zones RDPG1 (CAA65072; Petersen et al, 1996), oilseed rape pod (CAA90272; Jenkins et al, unpublished), turnip silique valve desiccation (CAD21651; Rodriguez-Gacio et al, 2004), Bell pepper fruit (BAE47457; Ogasawara S and Nakajima T, unpublished), tomato fruit TFPG (CAA32235; Bird et al, 1988), tomato pistil (AAC70951; Hong and Tucker, 2000), tomato abscission zones TAPG1, 2, 4, 5 (AAC28903, AAB09575, AAB09576, AAC28906; Hong and Tucker, 1998), tomato wound leaf (AAD17250; Bergey et al, 1999) and tomato seed (AAF61444; Sitrit et al, 1999).

### 1.1.6 Total RNA isolation, cDNA synthesis and quantitative PCR analysis:

Total RNA was isolated using the RNeasy Plant Mini Kit (Qiagen, Hilden, Germany). Between 50 and 100 mg of each tissue (anthers, fruit, flower abscission zone and leaf abscission zone) was used per RNA isolation reaction. Only 1µg of total RNA was used per sample for the synthesis of cDNA, after DNase I treatment (Boehringer Manheim). First strand cDNA template was synthesized using random hexamers as primers and Multiscribe™ Reverse Transcriptase (Applied Biosystems). The quasi complete coding sequence of ORF4 was amplified to study the intron splicing using the forward primer: TAGCTCCAAAGCTATCCACAT, located on the first exon, starting 47 nucleotides down-stream the start codon and the reverse primer: TGGAGAATGTGAAATTGTTAGG, located on the last exon, stopping 100 nucleotides up-stream the stop codon. Quasi-complete CDS of ORF4 was amplified with standard PCR reaction (55°C annealing temperature and 35 cycles)

Real-time experiments were conducted in an iCycler MyiQ detection system (Bio-Rad), using the SYBR green PCR master mix kit (Applied Biosystems). Primer sequences were: forward primer 5'-TTTTGCCATTGCCATTGATA-3', reverse primer 5'-TGTGGTGTCCCAGAACAAGA-3' (ORF4); and forward primer 5'-ACCACTTTCCGATCTCCTCTC-3', reverse primer 5'-ACCAGCAAATCCAGCCTTCAC-3' (β-actin). Relative quantification of the ORF4 transcript level was calculated with the internal β-actin control by applying the 2^{-ΔCT} formula. Purity of the PCR products was verified with the melting curves. The reactions were done in duplo. PCR controls were performed in absence of added reverse transcriptase to ensure RNA samples were free of DNA contamination.

### 2. Results

### 2.1 Microscopic observation of the ps-2 phenotype

Transverse section of anthers of wild-type (Moneymaker) and mutant (ps-2ABL) were prepared and stained with toluidine blue to identify the stage at which anther development/dehiscence in the mutant is blocked. Longitudinal sections of anther cones at anthesis were prepared for electron microscopy. No difference in developmental stages was visible until anthesis. At late Flower Bud, crystals were observed in the septum cells of mutant and wild-type, and pollen development appeared normal (Figure 1a). Breakage of the septum did occur in the mutant at similar stage than the wild type. However at anthesis the mutant stomium did not degenerate and the pollen remained in the anthers (Figure 1-b,c). In addition, endothecial thickening did not occur in the mutant and therefore the epidermal cells lacked rigidity at anthesis stage in order to create a breaking force on the stomium.

### 2.2 Physical mapping and candidate gene identification

We have previously mapped the *ps-2* locus to an interval of 1.65cM defined by COS derived CAPS markers T0958 and T0635 on the short arm of Chromosome 4 (Gorguet et al, 2006). A physical map for the *ps-2* locus region was built using the Heinz BAC library. The library was screened by PCR amplification with the closest markers relative to the *ps-2* locus and by computational means using the sequences of those markers. Positive BACs were then anchored to the genetic map by converting BAC ends into PCR markers (Figure 2) and by screening these markers in the recombinant population. BAC fingerprints were also compared to evaluate the overlapping of BACs from the same contig and to verify whether BACs were part of the same contig. The closest BAC end relative to the *ps-2* locus was then used for a second round of screening. Eventually, the entire contig spanned 1.70 cM (32 recombinants) from COS derived CAPS marker T1070 to BAC end 15N23-T (Figure 2). BAC 143M15 spanned the *ps-2* locus and was therefore sequenced.

In order to identify the genes present in BAC clone 143M15, the BAC DNA sequence was scanned against the Tomato SGN Unigene database, using a BLASTN interface, and against the *Arabidopsis* gene models database, using a TBLASTX interface. Two tomato coding sequences and five *Arabidopsis* genes matched the BAC clone sequence (two of them matching the two tomato coding sequences; Table 2). The five candidate genes were named ORF1 to ORF5. In addition, six genes of the retrotransposon family were also identified but were not taken into account in the further study. The positions of the five corresponding *Arabidopsis* genes were not contiguous in the *Arabidopsis* genome which highlighted the absence of synteny between the two species for that region. Moreover none of them was homolog to one of the functional male sterility genes identified in *Arabidopsis* (listed in Gorguet et al., 2006).

**Table 2: Identification of candidate gene based on Arabidopsis gene models**

| Gene | Tomato | *Arabidopsis* | | | | | |
|---|---|---|---|---|---|---|---|
| | Unigenes Acc. no. | Accession no. | Gene function | | AGI coordinates (bases) | TBLASTX | |
| | | | | Chr. | | *E* value | Score |
| ORF1 | U323899 | AF014399 | magnesium-chelatase | 1 | 2696415-2700961 | 0 | 214 |
| ORF2 | | NM112785 | transcriptional factor B3 family | 3 | 6548875-6551847 | 3E-21 | 105 |
| ORF3 | U317249 | AF326883 | remorin family protein | 2 | 17477944-17480014 | 9E-37 | 110 |
| ORF4 | | NM111676 | polygalacturonase | 3 | 2541012-2543438 | 1E-79 | 108 |
| ORF5 | | AB017502 | glycosyl hydrolase family 3 | 5 | 7107378-7111311 | 0 | 276 |

In order to localize the candidate genes in the high resolution map, we converted the putative genes sequences (or the sequences nearby) into PCR markers and mapped them in the recombinant population. Every candidate genes mapped at different positions in the high resolution linkage map and therefore we could easily identify the likeliest candidate for *ps-2* based on their positions in the genetic map. ORF4, a putative polygalacturonase gene, mapped the closest to the *ps-2* locus (Figure 2). Subsequently the putative introns and exons were identified using the FGENSH software of Softberry. The candidate gene ORF4 is composed of nine exons and eight introns, covering a genomic distance of 6716 nucleotides from putative start to stop codon, for a coding sequence of 1179 nucleotides. The SNP used to develop the PCR marker ORF4 was located in the second putative intron. We developed and mapped two extra PCR markers, one based on a deletion of 76bp in the first intron in the S. *pimpinellifolium* allele [ORF4(1)] and one based on an insertion of 38bp in the sixth intron in the *S. pimpinellifolium* allele [ORF4(2)]. The three PCR markers, ORF4(1), ORF4 and ORF4(2) mapped at an interval of one recombinant between each other, indicating that at least two recombinations had occurred within the candidate gene ORF4 in the recombinant population (Figure 2). ORF4(2) co-segregated with the *ps-2* locus on the high resolution map.

### 2.3 Mutation in ORF4 and molecular marker development

To consolidate the hypothesis that ORF4 corresponds to the *ps-2* gene, we searched for sequence alterations in the *ps*-2ABL allele. The entire 9kb, from the putative promoter to the putative terminator of ORF4 was sequenced on *cv*. Moneymaker and *ps*-2ABL. One single mutation was identified on the last nucleotide of the fifth putative exon of ORF4, in which the nucleotide Guanine was replaced by Cytosine. To test the association between the identified SNP in ORF4 and the *ps-2* trait, we developed a molecular marker based on that SNP, in such a way that the *ps*-2ABL allele and the wild type allele in *S. lycopersicum* could easily be differentiated on gel. This marker was tested on a set of 176 ABLs among which eight were *ps-2*/*ps-2.* These seven functionally male sterile plants showed the same marker pattern, distinct from the other ABLs, which confirm that the SNP is present in the other *ps-2* lines tested. This marker can now easily be used for molecular assisted introduction of the *ps-2* trait into modem tomato lines.

### 2.4 Alternative intron splicing

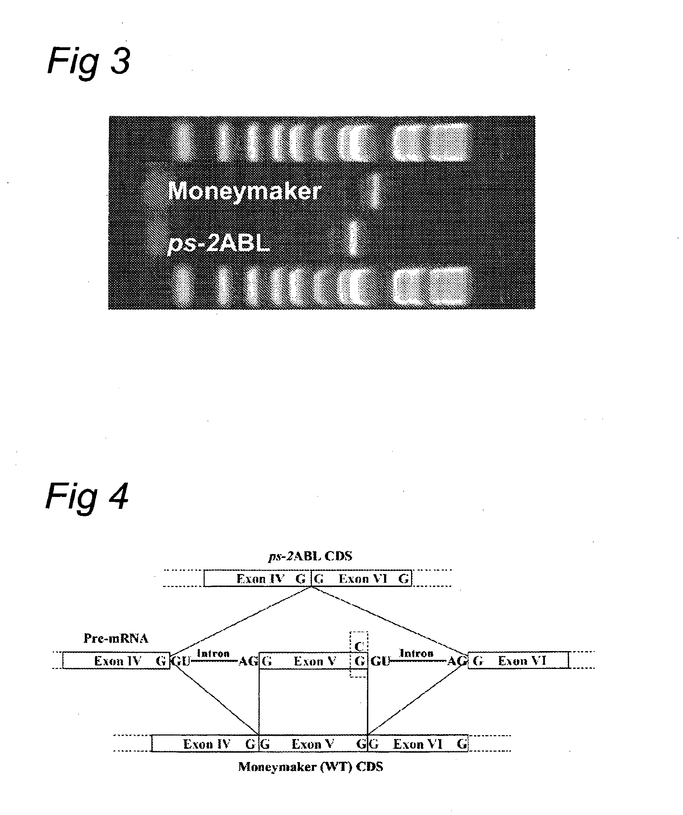

Because the sequence mutation in the *ps*-2ABL allele of ORF4 is located in one of the intron recognition splice sites, we hypothesized that this mutation could affect the pre-mRNA splicing of the gene. In order to verify this hypothesis we designed primers to amplify the quasi full-length cDNA clone of ORF4 (1032nt out of 1179nt): The forward primer was designed on the first exon of ORF4 and the reverse primer on the last exon (Table 3). RT-PCR was performed on cDNA made from RNA of anther cones at post-anthesis from *ps*-2ABL and cv. Moneymaker. The observed amplified product for *cv*. Moneymaker was of the expected size (1032bp), which was confirmed by sequencing. The amplified product of *ps*-2ABL was significantly smaller than the Moneymaker product which suggested an alteration in the introns splicing (Figure 3). The sequencing of the fragment amplified on cDNA of *ps*-2ABL showed that the fifth exon, on which the SNP is present, was skipped in the cDNA sequence. This exon was removed together with the two flanking introns during the pre-mRNA maturation process (Figure 4). The wild type 5' sequence of this exon-intron splice junction is CAG/GTATCG, which is identical to one of the splice junction sequence identified in *Solanum tuberosum* (Brown, 1986). The mutation found in the *ps-2ABL* allele induces the following sequence: CAC/GTACG, which is not present in the list of intron splicing recognition sites. The absence of the fifth exon in the mature mRNA represents a deletion of 208 nucleotides, which induces a frame-shift on the remaining coding sequence down-stream. This frame-shift causes a premature termination of translation after 14aa due to a newly framed stop codon. The complete putative mutated protein is therefore 154 aa long in comparison to 392 aa for the wild type protein, and is likely to be non-functional.

### 2.5 ORF4 sequence analysis

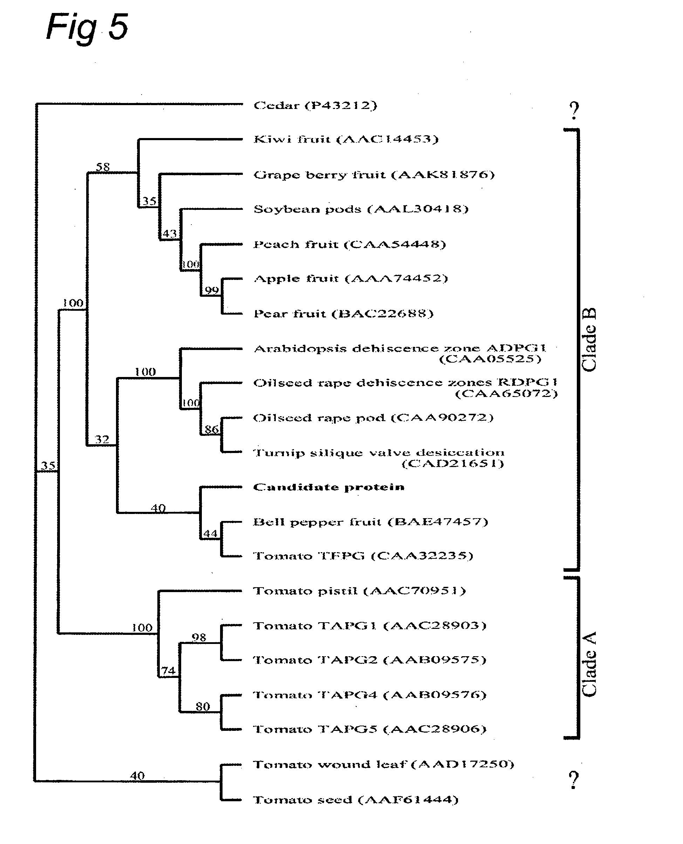

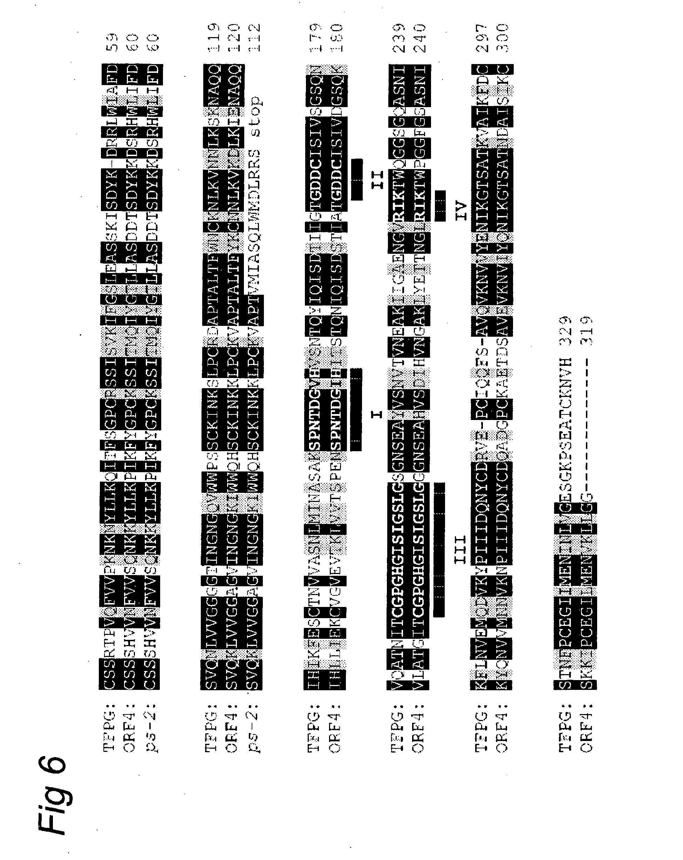

A BLAST search with the putative candidate protein sequence of ORF4, in the protein database of NCBI, resulted in a list of PG proteins from several plants. The identified proteins have functions in fruit ripening and siliques/pods dehiscence. Among them, the ADPG1 protein had been found to be expressed in the dehiscence zone of siliques of *Arabidopsis* as well as the dehiscence zone of anthers (Sander et al, 2001). Amino acids of the Pfam GH28 domain of the best BLAST hits were aligned together with the sequences of the candidate protein and the already known tomato PG proteins with identified functions, and one gymnosperm PG (cedar). A phylogenetic analysis was performed on the final alignment in order to place the candidate protein in one of the referenced PG clade. ORF4 was identified as a PG of clade B (Figure 5). Clade B is composed of all the cloned genes that encode PG expressed in fruit and dehiscence zone, as previously characterized by Hadfields and Bennett (1998). This was also observed in the present phylogenetic tree. TFPG, the only tomato PG known to be expressed in fruits, was also part of the same clade. Alignment of Pfam GH28 domains of ORF4 and TFPG is presented in Figure 6. ORF4 and TFPG have a similarity of 59% on the entire protein sequence.

The putative derived protein of ORF4 contains the four conserved domain characteristic of PG proteins, as presented by Rao et al (1996; Figure 6). The first conserved domain is located on the fifth exon, which is skipped in the mutant protein and the three others domains are located further on the sequence and therefore not in framed in the mutant sequence. Thus the mutant protein does not contain any of the four conserved domains that play a major role in the function of the protein. Analysis of the 2000 nucleotides up-stream the start codon of ORF4 revealed the presence of three Ethylene Responsive Elements (ERE; AWTTCAAA) at positions -667, -700 and -1955 relative to the start codon, one bZIP protein binding motif (TGACG) at -1632 and one G-box (CACGTG) at -1329. The presence of ERE motifs, bZIP protein binding motifs and G-box in the promoter sequence of ORF4 suggests that the transcription of ORF4 is stimulated by ethylene and jasmonate.

### 2.6 Expression of ORF4 in anther, fruit and other tissues

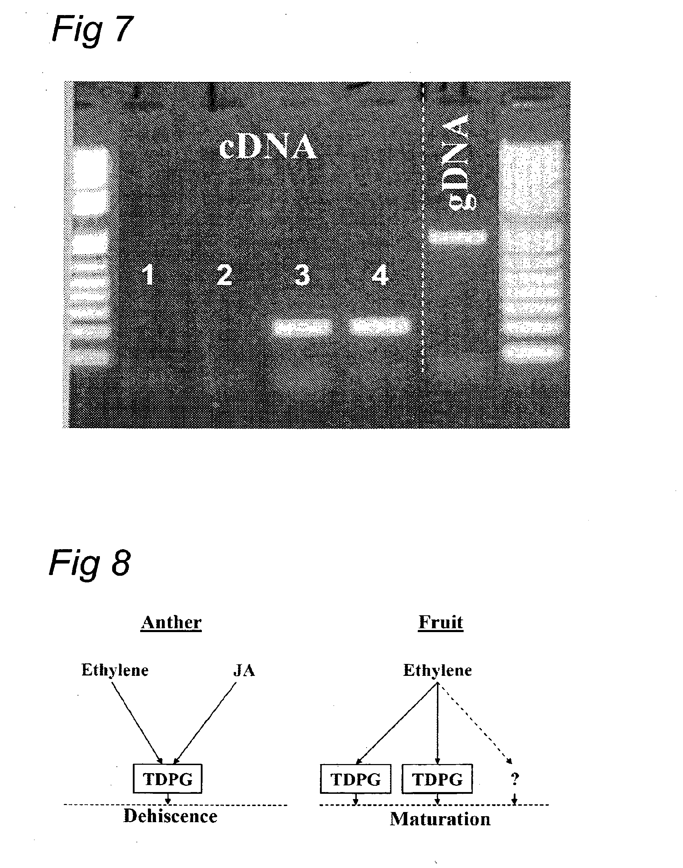

We tested the presence of ORF4 transcript in several tissues including abscission zones of leaf and flowers, mature fruits and anthers at anthesis. No ORF4 transcript was detected in the abscission zones (Figure 7). The presence of ORF4 transcript was confirmed in anthers as well as in the fruit. In order to study the evolution of the transcription level of ORF4 over different stages of anther and fruit development, we performed a quantitative expression analysis of ORF4 at four developmental stages of anthers: flower bud; pre-anthesis; anthesis; post-anthesis, and eight developmental stages of the fruit, from five dap (days after pollination) to 57 dap (mature fruit); 47 dap corresponded to breaker stage (data not shown). In anthers, the transcription level of ORF4 was tested on Moneymaker and *ps*-2ABL, and in the fruits only on Moneymaker. No ORF4 transcript was detected in the fruit before 37 dap. From 37 dap ORF4 transcript was detected and increased significantly over time to reach a maximum at mature stage (57dap). In anthers of Moneymaker, ORF4 transcripts were detected already at flower bud stage. At pre-anthesis the level of ORF4 transcript was similar to flower bud. ORF4 transcript accumulation increased at anthesis and reached a maximum at post-anthesis. In anthers of *ps*-2ABL*,* ORF4 transcript was also detected, except at flower bud stage. However this transcript level in *ps*-2ABL anthers was significantly lower than in Moneymaker anthers at anthesis and post-anthesis.

### 3. Discussion

The *ps-2* Advanced Breeding Line produces anthers that do not undergo dehiscence. In this study we showed that anther development/dehiscence of *ps*-2ABL is blocked in the ultimate phase. The stomium does not degenerate and the endothecium wall does not thicken. In absence of these two physiological changes, the anther remains closed and the epidermal cells lack the rigidity that could eventually break the stomium and liberate the pollen.

This phenotypic mutation is recessive and under the control of one single locus. In a previous study we fine mapped the *ps-2* gene on the short arm of Chromosome 4 (Gorguet et al, 2006). Here we report the isolation and functional characterization of the *ps-2* gene. We found that the *ps-2* phenotype is the result of a single nucleotide mutation in a polygalacturonase gene unknown to date, composed of nine exons. This single nucleotide mutation is located on the last nucleotide of the 3' end of the fifth exon, affecting the intron splicing recognition site, which is changed from CAG/GTATCG to CAC/GTATCG (exon 3'/intron 5'). Though the Cytosine base is met in 11% of the intron splice sites at this specific position in plants, the combination "CAC" at the exon 3'end has never been detected in any splice site in plants (Brown 1986). The 5^{th} exon is spliced out together with the two flanking introns. Analysis of *Arabidopsis* mutants with mutations around splice sites has revealed several examples of exon skipping in plant splicing (reviewed by Brown and Simpson, 1998). Most of these mutations are located in the intron part of the recognition splice sites. To our knowledge, in plants, the only mutant showing exon skipping due to a mutation in the exon part of a recognition splice site, to date, was the *spy-1* mutant in *Arabidopsis* (Jacobsen et al, 1996) in which the CAG/GTTTGA (exon 3'/intron 5') recognition splice site at the end of the eight exon was mutated into CAA/GTTTGA. The exon skipping observed in the mutated allele of ORF4 induces a frame-shift in the rest of the sequence, which has for consequence to create an early stop codon 14 aa further. The complete mutant protein is therefore 154 aa long in comparison to 392 aa long for the wild type, and do not contain any of the four domains characteristic of PGs.

### 3.1 ps-2 is a PG of clade B

The isolated gene responsible for the *ps-2* phenotype is a PG unknown to date. We propose the acronym DPG, standing for Dehiscence PolyGalacturonase, or in the case of the tomato gene TDPG, standing for Tomato Dehiscence PolyGalacturonase. Phylogenetic analysis of TDPG revealed a close similarity with PG of clade B as defined by Hadfield and Bennett (1998). Clade B is here composed of fruit PGs, among them the Tomato Fruit PG, as well as silique or pod dehiscence PGs. Other tomato PGs cluster in different clades. In accordance to the assertion that the divergence of PG gene families occurred prior to the separation of the angiosperm species (Hadfield and Bennett, 1998), TDPG is here more closely related to genes of the same clade, from other species, than to tomato PGs from other clades. Most of the other tomatoes PGs are related to abscission (TAPG). In our study, expression of TDPG was not detected in flower and leaf abscission zones. However, in addition to anther tissues, we detected mRNA transcript of TDPG in fruits.

### 3.2 TDPG transcript accumulation increases along with the development of the anthers

We measured the relative level of TDPG transcript at different stages in anthers. TDPG transcript is already detected in anthers of Moneymaker at flower bud stage. The transcript level increases over stages to reach a maximum at post-anthesis, when anthers are dehisced. This increase of TDPG transcript accumulation is parallel to the septum and stomium degeneration as well as the thickening of the endothecial cell wall observed in the anthers. TDPG transcript level in *ps*-2ABL anthers was detected from pre-anthesis on, but the transcript level remained very low in comparison to Moneymaker, at anthesis and post-anthesis. Very likely the mutant mRNA is recognized as non-sense and degraded by Nonsense-mediated mRNA decay (NMD). NMD functions as a quality control mechanism to eliminate abnormal transcripts (Lejeune and Maquat 2005).

### 3.2 TDPG is likely under the control of Ethylene and Jasmonate:

The presence of ethylene and jasmonate responsive elements in the promoter region of TDPG suggests that the transcription of DPG genes could be induced by both hormones. Ethylene has already been involved in the timing of anther dehiscence in tobacco (Rieu et al, 2003). More recently, in petunia, it has been shown that ethylene regulates the synchronization of anther dehiscence with flower opening (Wang and Kumar, 2006). In addition, many studies already identified jasmonate as a key compound in the process and timing of anther dehiscence. Several mutants in JA biosynthetic enzymes have been identified for the study of this phenomenon. Scott et al (2004) have suggested that ethylene and JA may act redundantly in the control of anther dehiscence, which would explain why *Arabidopsis* mutants such as *dde-I,* which cannot synthesize JA within the stamens, or the ethylene insensitive *Tetr* mutant, eventually undergo anther dehiscence.

In the present study we showed that anthers of *ps*-2ABL remain indehiscent, in contrast to delayed dehiscence in the other mutants, which strengthen the hypothesis that DPG acts down-stream of Ethylene and JA in the control of anther dehiscence and that DPG is the main actor of this process.

### 3.3 TDPG may also play a role in tomato fruit ripening:

TFPG, the only tomato fruit polygalacturonase identified to date, has been characterized as one of the main actors in the process of fruit softening. Anti-sense repression of TFPG has lead to the production of tomato fruit with longer shelf life but the fruits did undergo ripening indicating that other actors also play a relevant role in the process of fruit softening (Smith et al, 1988). DPGs may well be one of these actors by contributing to the fruit cell wall degradation. TDPG transcript was detected in the late stages of fruit development in Moneymaker (Figure 8). Maximum transcripts were found at mature stage (57dap). Similarly TFPG has been detected only at ripening stages, starting at mature green or at breaker stage (Thompson et al, 1999; Eriksson et al, 2004). The level of TFPG was also found to increase over time from breaker stage to mature fruit, in *cv*. AC and Liberto (Thompson et al, 1999).

In tomato, although the regulation of PG mRNA accumulation by ethylene remained for a long time ambiguous, it has been demonstrated that TFPG accumulation was ethylene dependent in the process of fruit ripening (Sitrit and Bennett, 1998). In accordance ERE motifs have been found in the promoter of TFPG (Montgomery et al, 1993). Ethylene is presented as the major plant hormone in the control of fruit ripening (reviewed by Giovannoni, 2004). Expression of anti-sense RNA to the rate limiting enzyme in the biosynthetic pathway of ethylene inhibits fruit ripening in tomato and as consequence down regulates the production of TFPG (Oeller et al, 1991; Sitrit and Bennett, 1998).

Earlier in this study we suggested that DPG was under the control of ethylene due to the presence of ERE motifs in the promoter sequence. Repression of ethylene in the tomato fruit is likely to inhibit both TFPG and TDPG and therefore prevents completely the process of fruit ripening. A simplified model for the hormonal control of DPG in anther dehiscence and fruit maturation is presented in Figure 8.

It is not clear whether the role of DPG in fruit maturation and shelf life is of similar importance than TFPG. The comparison between *ps*-2ABL after manual opening of the anthers and Moneymaker or any other normal tomato line is unreliable due to the difference of genetic background. Knock out of DPG by anti-sense RNA or RNAi in a normal tomato line could answer to that question. Time between hand pollination to mature fruit stage and fruit shelf life could be measured and compared to the untransformed control in order to evaluate the effect of DPG in the fruit. Knock out of homologs of TDPG in other plants species is also of valuable interest to verify whether the control of anther dehiscence it its ultimate phase is conserved among species.

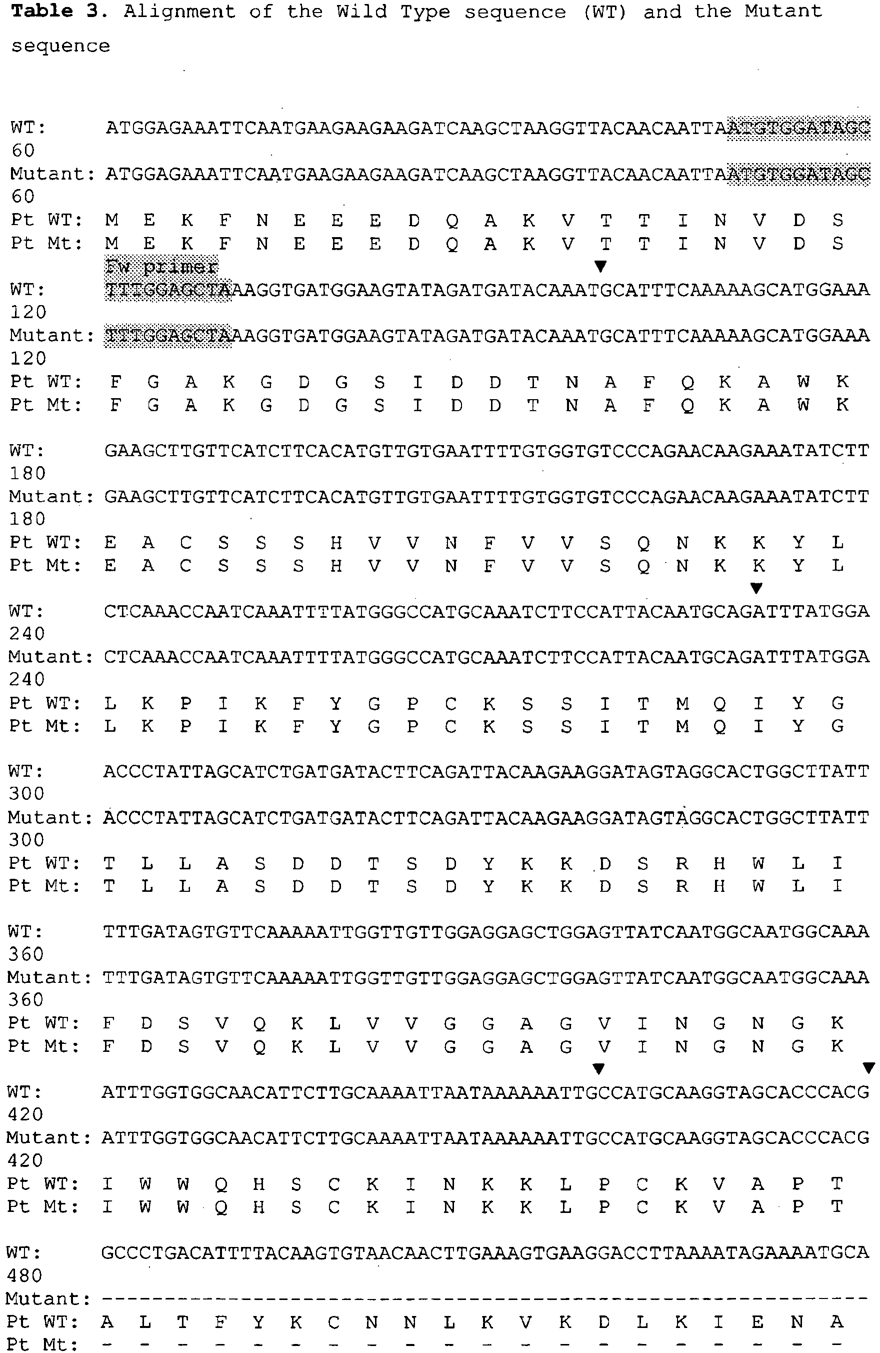

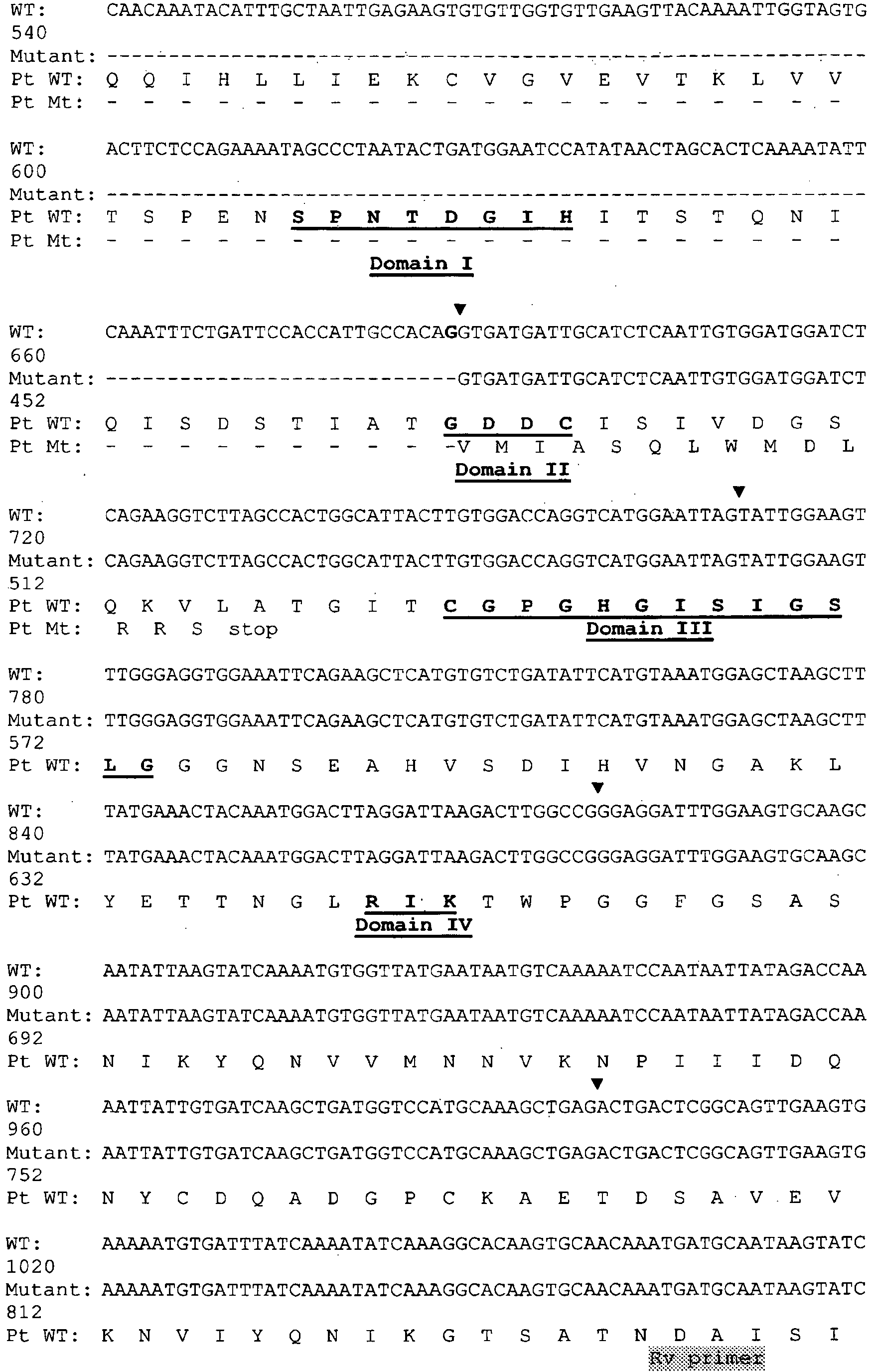

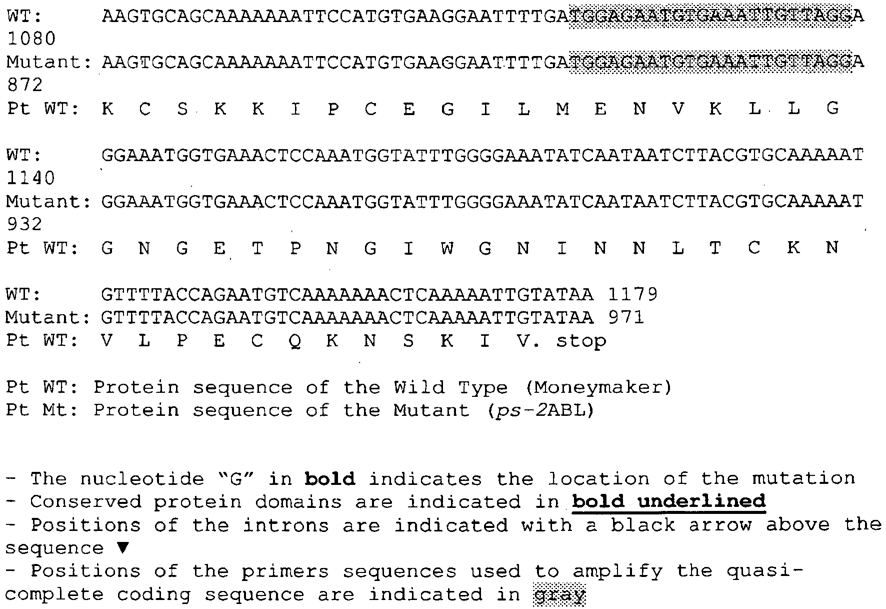

### References

Atkinson R G and Gardner R C (1993) A polygalacturonase gene from kiwifruit (Actinidia deliciosa) Plant Physiol. 103:669-670
Atkinson R G (1994) A cDNA clone for endopolygalacturonase from apple. Plant Physiol. 105: 1437-1438
Bergey D R, Orozco-Cardenas M, de Moura D S, Ryan C A (1999) A wound- and systemin-inducible polygalacturonase in tomato leaves Proc. Natl. Acad. Sci. U.S.A. 96 (4), 1756-1760 (1999)
Bird C R, Smith C J, Ray J A, Moureau P, Bevan M W, Bird A S, Hughes S, Morris P C, Grierson D, Schuch W (1988) The tomato polygalacturonase gene and ripening-specific expression in transgenic plants Plant Mol. Biol. 11: 651-662
Brown J W S (1986) A catalogue of splice junction and putative branch point sequences from plant introns. Nucleic acids research 14: 9549-9559
Brown J W S and Simpson C G (1998) Splice site selection in plant pre-mRNA splicing. Annual review of plant physiology and plant molecular biology 49:77-95 Brugmans B, Hutten R G B, Rookmaker A N O, Visser R G F, Van Eck H J (2006) Exploitation of a marker dense linkage map of potato for positional cloning of a wart disease resistance gene. Theoretical and Applied Genetics 112:269-277
Budiman M A, Mao L, Wood T C, Wing R A (2000) A deep-coverage tomato BAC library and prospects toward development of an STC framework for genome sequencing. Genome Res 10:129-136
Christiansen L C, Dal Degan F, Ulvskov P, Borkhardt B (2002) Examination of the dehiscence zone in soybean pods and isolation of a dehiscence-related endopolygalacturonase gene Plant Cell Environ. 25:479-490
Colbert T, Till BJ, Tompa R, Reynolds S, Steine MN, Yeung AT, McCallum CM, Comai L, Henikoff S. High-throughput screening for induced point mutations. Plant Physiol. 2001 Jun;126(2):480-4
Dornelas M, van Lammeren A, and Kreis M (2000). Arabidopsis thaliana SHAGGY-related protein kinases (AtSK11 and 12) function in perianth and gynoecium development. Plant Journal 21: 419-429
Dubald M, Barakate A, Mandaron P, Mache R (1993) The ubiquitous presence of exopolygalacturonase in maize suggests a fundamental cellular function for this enzyme. The plant journal 4: 781-791
Gorguet B, Schipper D, van Heusden A W, Lindhout P (2006) High resolution fine mapping of ps-2, a mutated gene conferring functional male sterility in tomato due to non-dehiscent anthers. Theoretical and Applied Genetics. 113:1437-1448
Hadfield K A, Bennett A B (1998) Polygalacturonases: Many genes in search of a function. Plant physiology 117: 337-343
Higgins D, Thompson J, Gibson T, Thompson J D, Higgins D G, Gibson T J (1994) CLUSTAL W: improving the sensitivity of progressive multiple sequence alignment through sequence weighting, position-specific gap penalties and weight matrix choice. Nucleic Acids Res. 22:4673-4680
Hong S B and Tucker M L (1998) Genomic organization of six tomato polygalacturonases and 5' upstream sequence identity with tap1 and win2 genes Mol. Gen. Genet. 258: 479-487
Hong S-B and Tucker M L, (2000) Molecular characterization of a tomato polygalacturonase gene abundantly expressed in the upper third of pistils from opened and unopened flowers. Plant Cell Reports 19: 680-683
Huala E, Dickerman A W, Garcia-Hernandez M, Weems D, Reiser L, LaFond F, Hanley D, Kiphart D, Zhuang M, Huang W, Mueller L A, Bhattacharyya D, Bhaya D, Sobral B W, Beavis W, Meinke D W, Town C D, Somerville C, Rhee S Y (2001) The Arabidopsis Information Resource (TAIR): a comprehensive database and web-based information retrieval, analysis, and visualization system for a model plant. Nucleic Acids Res. 29:102-105
Jacobsen S E, Binkowski K A, Olszewski N E (1996) SPINDLY, a tetratricopeptide repeat protein involved in gibberellin signal transduction in Arabidopsis. Proceedings of the National Academy of Sciences of the United States of America 93: 9292-9296 Jenkins E S, Paul W, Coupe S A, Bell S, Davies L, Roberts J A. Characterization and expression of an mRNA encoding polygalacturonase during pod development in oilseed rape (Brassica napus L.) Unpublished
Keijzer C J (1987) The processes of anther dehiscence and pollen dispersal. I. The opening mechanism of longitudinally dehiscing anthers. New Phytologist 105: 487-498 Lejeune F, Maquat L E (2005) Mechanistic links between nonsense-mediated mRNA decay and pre-mRNA splicing in mammalian cells. Current Opinion in Cell Biology 17: 309-315
Lester D R, Speirs J, Orr G, Brady C J (1994) Peach (Prunus persica) endopolygalacturonase cDNA isolation and mRNA analysis in melting and nonmelting peach cultivars Plant Physiol. 105: 225-231
Markovic O, Janecek S (2001) Pectin degrading glycoside hydrolases of family 28: sequence-structural features, specificities and evolution. Protein engineering 14: 615-631
McCallum CM, Comai L, Greene EA, Henikoff S. Targeted screening for induced mutations. Nat Biotechnol. 2000 Apr;18(4):455-7
McCallum CM, Comai L, Greene EA, Henikoff S. Targeting induced local lesions IN genomes (TILLING) for plant functional genomics. Plant Physiol. 2000a Jun;123(2):439-42
Montgomery J, Pollard V, Deikman J, Fischer R L (1993) Positive and negative regulatory regions control the spatial-distribution of polygalacturonase transcription in tomato fruit pericarp. The plant cell 5: 1049-1062
Mueller L A, Solow T H, Taylor N, Skwarecki B, Buels R, Binns J, Lin C, Wright M H, Ahrens R, Wang, Y, Herbst E V, Keyder E R, Menda N, Zamir D, Tanksley S D (2005) The SOL Genomics Network. A comparative resource for Solanaceae biology and beyond. Plant Physiol 138:1310-1317
Nunan K J, Davies C, Robinson S P and Fincher G B (2001) Expression patterns of cell wall-modifying enzymes during grape berry development. Planta 214:257-264
Oeller P W, Min-Wong L, Taylor L P, Pike D A, Theologis A (1991) Reversible inhibition of tomato fruit senescence by antisense RNA. Science 254: 437-439 Ogasawara S and Nakajima T. A gene coding for bell pepper endo-polygalacturonase is expressed during fruit ripening. Unpublished
Petersen M, Sander L, Child R, van Onckelen H, Ulvskov P, Borkhardt B (1996) Isolation and characterisation of a pod dehiscence zone-specific polygalacturonase from Brassica napus. Plant Mol. Biol. 31: 517-527
Rao M N, Kembhavi A A, Pant A (1996) Implication of tryptophan and histidine in the active site of endo-polygalacturonase from Aspergillus ustus: elucidation of the reaction mechanism. Biochim. Biophys. Acta 1296: 167-173
Rieu I, Wolters-Arts M, Derksen J, Mariani C, Weterings K (2003) Ethylene regulates the timing of anther dehiscence in tobacco. Planta 217: 131-137
Rodriguez-Gacio M, Nicolas C, Matilla A (2004) Cloning and analysis of a cDNA encoding an endo-polygalacturonase expressed during the desiccation period of the silique-valves of turnip-tops (Brassica rapa L . cv. Rapa) Plant Physiol. 161: 229-236 Rouster J, Leah R, Mundy J, Cameron-Mills V (1997) Identification of a methyl jasmonate-responsive region in the promoter of a lipoxygenase 1 gene expressed in barley grain. The plant journal 11: 513-523
Sander L, Child R, Ulvskov P, Albrechtsen M, Borkhardt B (2001) Analysis of a dehiscence zone endo-polygalacturonase in oilseed rape (Brassica napus) and Arabidopsis thaliana: evidence for roles in cell separation in dehiscence and abscission zones, and in stylar tissues during pollen tube growth. Plant Mol. Biol. 46: 469-479
Scott R J, Spielman M, Dickinson H G (2004) Stamen structure and function. The plant cell 16: S40-S60
Sitrit Y, Hadfield K A, Bennett A B, Bradford K J, Downie A B (1999) Expression of a polygalacturonase associated with tomato seed germination Plant Physiol. 121: 419-428
Smith C J S, Watson C F, Ray J, Bird C R, Morris P C, Schuch W, Grierson D (1988) Anti-sense RNA inhibition of polygalacturonase gene expression in transgenic tomatoes. Nature 334: 724-726
Swofford D L (2002) PAUP*. Phylogenetic Analysis Using Parsimony (*and Other Methods). Version 4. Sinauer Associates, Sunderland, Massachusetts
Torki M, Mandaron P, Mache R, Falconet D (2000) Characterization of a ubiquitous expressed gene family encoding polygalacturonase in Arabidopsis thaliana. Gene 242:427-436
Wang Y, Kumar P P (2006) Characterization of two ethylene receptors PhERS1 and PhETR2 from petunia: PhETR2 regulates timing of anther dehiscence. Journal of Experimental Botany doi:10.1093/jxb/er1229

## Claims

1. A nucleic acid molecule comprising a nucleotide sequence encoding a *ps*-2-allele, wherein the *ps*-2-allele is an allele that has a C, A or T as last nucleotide of the 3' end of the fifth exon of the dehiscence polygalacturonase gene, wherein the dehiscence polygalacturonase is a polypeptide with dehiscence polygalacturonase activity, wherein the polypeptide comprises an amino acid sequence that has at least 90% sequence identity with the amino acid sequence of SEQ ID NO: 2, or a nucleic acid molecule comprising a nucleotide sequence of at least 10 contiguous nucleotides from SEQ ID NO: 1 and comprising a C, A or T at position 3772 of SEQ ID NO: 1.

2. A nucleic acid molecule according to claim 1, wherein the polypeptide with dehiscence polygalacturonase activity is obtainable from a species within the genus *Solanum.*

3. A nucleic acid molecule according to claim 2, wherein the polypeptide with dehiscence polygalacturonase activity is obtainable from a species within the *Solanum lycopersicum* complex or from *Solanum melongena.*

4. A method for detecting, isolating, amplifying and/or analysing a dehiscence polygalacturonase allele in a plant, the method comprising the step of providing a sample comprising nucleic acids of the plant and hybridising the nucleic acids of the plant with a nucleic acid molecule comprising a nucleotide sequence of at least 10 contiguous nucleotides from a nucleotide sequence as defined in claim 1.

5. A method according to claim 4, wherein the dehiscence polygalacturonase allele is a *ps*-2-allele, wherein the *ps*-2-allele is an allele that has a C, A or T as last nucleotide of the 3' end of the fifth exon of the dehiscence polygalacturonase gene, preferably the allele has a C as last nucleotide of the 3' end of the fifth exon of the dehiscence polygalacturonase gene.

6. Use of a nucleic acid molecule comprising a nucleotide sequence of at least 10 contiguous nucleotides from a nucleotide sequence as defined in any one of claims 1 - 3 in marker-assisted breeding.

7. A use according to claim 6, wherein the nucleic acid molecule 5 is used in marker-assisted breeding to produce plants with a positional sterile phenotype due to non-dehiscent anthers and/or to alter fruit ripening.

8. A method for producing a plant with a mutation in a dehiscence polygalacturonase allele, wherein the method comprises the steps of:
a) mutagenising seeds of a plant complex;
b) growing plants of the mutagenised seeds obtained in a); and,
c) screening plants obtained in b) for the presence of a mutation a C, A or T in the last nucleotide of the fifth exon of the dehiscence polygalacturonase gene, wherein the dehiscence polygalacturonase is a polypeptide with dehiscence polygalacturonase activity, wherein the polypeptide comprises an amino acid sequence that has at least 90% sequence identity with the amino acid sequence of SEQ ID NO: 2.

9. A method according to claim 8, wherein the method is a method for producing a plant with a positional sterile phenotype due to non-dehiscent anthers and/or a plant with altered fruit ripening.

10. A method according to claim 9, wherein the mutation in the dehiscence polygalacturonase allele causes the allele to have a C as last nucleotide of the 3' end of the fifth exon of the dehiscence polygalacturonase gene.

11. A method for producing a transgenic plant with non-dehiscent anthers and/or altered fruit ripening, wherein the method comprises the step of transforming a plant cell with a nucleic acid construct comprising a nucleotide sequence encoding a *ps-2-*allele, wherein the *ps*-2-allele is an allele that has a C, A or T as last nucleotide of the 3' end of the fifth exon of the dehiscence polygalacturonase gene, wherein the dehiscence polygalacturonase is a polypeptide with dehiscence polygalacturonase activity, wherein the polypeptide comprises an amino acid sequence that has at least 90% sequence identity with the amino acid sequence of SEQ ID NO: 2 , or a complement thereof, wherein presence of the nucleic acid construct in a cell of the plant reduces expression of dehiscence polygalacturonase activity to a level that effects positional sterility and non-dehiscent anthers and/or altered fruit ripening.

12. A method according to claim 11, wherein nucleic acid construct is a construct for homologous recombination and wherein the nucleotide sequence comprises a C, A or T in the last nucleotide of the fifth exon of the dehiscence polygalacturonase gene that reduces expression of dehiscence polygalacturonase activity to a level that effects positional sterility and non-dehiscent anthers and/or altered fruit ripening.

13. A method for detecting a plant with a mutation in a dehiscence polygalacturonase allele, wherein the method comprises screening plants for the presence of a C, A or T in the last nucleotide of the fifth exon of the dehiscence polygalacturonase gene, wherein the dehiscence polygalacturonase is a polypeptide with dehiscence polygalacturonase activity, wherein the polypeptide comprises an amino acid sequence that has at least 90% sequence identity with the amino acid sequence of SEQ ID NO: 2.

## Patentansprüche

1. Nukleinsäuremolekül umfassend eine ein *ps*-2-Allel kodierende Nukleotidsequenz, wobei das *ps*-2-Allel ein Allel ist, das als letztes Nukleotid des 3'-Endes des fünften Exons des Dehiszenz-Polygalacturonasegens ein C, A oder T aufweist, wobei die Dehiszenz-Polygalacturonase ein Polypeptid mit Dehiszenz-Polygalacturonaseaktivität ist, wobei das Polypeptid eine Aminosäuresequenz umfasst, die mindestens 90% Sequenzidentität mit der Aminosäuresequenz von SEQ ID NO: 2 aufweist, oder ein Nukleinsäuremolekül umfassend eine Nukleotidsequenz von mindestens 10 benachbarten Nukleotiden aus SEQ ID NO: 1 sowie umfassend ein C, A oder T an der Position 3772 der SEQ ID NO: 1.

2. Nukleinsäuremolekül nach Anspruch 1, wobei das Polypeptid mit Dehiszenz-Polygalacturonaseaktivität aus einer Spezies der Gattung *Solanum* erhältlich ist.

3. Nukleinsäuremolekül nach Anspruch 2, wobei das Polypeptid mit Dehiszenz-Polygalacturonaseaktivität aus einer Spezies des *Solanum Lycopersicum-*Komplexes oder aus *Solanum melongena* erhältlich ist.

4. Verfahren zum Nachweisen, Isolieren, Verstärken und/oder Analysieren eines Dehiszenz-Polygalacturonase-Allels in einer Pflanze, wobei das Verfahren den Schritt des Bereitstellens einer Probe umfassend Nukleinsäuren der Pflanze und das Hybridisieren der Nukleinsäuren der Pflanze mit einem Nukleinsäuremolekül umfassend eine Nukleotidsequenz von mindestens 10 benachbarten Nukleotiden aus einer in Anspruch 1 definierten Nukleotidsequenz umfasst.

5. Verfahren nach Anspruch 4, wobei das Dehiszenz-Polygalacturonase-Allel ein *ps-2-*Allel ist, wobei das *ps*-2-Allel ein Allel ist, das als letztes Nukleotid des 3'-Endes des fünften Exons des Dehiszenz-Polygalacturonasegens ein C, A oder T aufweist, vorzugsweise weist das Allel als letztes Nukleotid des 3'-Endes des fünften Exons des Dehiszenz-Polygalacturonasegens ein C auf.

6. Verwendung eines Nukleinsäuremoleküls umfassend eine Nukleotidsequenz von mindestens 10 benachbarten Nukleotiden aus einer in einem der Ansprüche 1 bis 3 definierten Nukleotidsequenz bei markergestützter Züchtung.

7. Verwendung nach Anspruch 6, wobei das Nukleinsäuremolekül bei markergestützter Züchtung verwendet wird, um Pflanzen mit einem positionellen sterilen Phänotyp aufgrund nicht dehiszenter Antheren zu erzeugen und/oder Fruchtreife zu verändern.

8. Verfahren zum Erzeugen einer Pflanze mit einer Mutation in einem Dehiszenz-Polygalacturonase-Allel, wobei das Verfahren die Schritte umfasst:
a) Mutagenisieren von Samen eines Pflanzenkomplexes;
b) Anbauen von Pflanzen der in a) erhaltenen mutagenisierten Samen; und
c) Überprüfen von in b) erhaltenen Pflanzen auf das Vorhandensein eines C, A oder T im letzten Nukleotid des fünften Exons des Dehiszenz-Polygalacturonasegens, wobei die Dehiszenz-Polygalacturonase ein Polypeptid mit Dehiszenz-Polygalacturonaseaktivität ist, wobei das Polypeptid eine Aminosäuresequenz umfasst, die mindestens 90% Sequenzidentität mit der Aminosäuresequenz von SEQ ID NO: 2 aufweist.

9. Verfahren nach Anspruch 8, wobei das Verfahren ein Verfahren zum Erzeugen einer Pflanze mit einem positionellen sterilen Phänotyp aufgrund nicht dehiszenter Anthe-ren und/oder einer Pflanze mit veränderter Fruchtreife ist.

10. Verfahren nach Anspruch 9, wobei die Mutation in dem Dehiszenz-Polygalacturonase-Allel dafür sorgt, dass das Allel als letztes Nukleotid des 3'-Endes des fünften Exons des Dehiszenz-Polygalacturonasegens ein C aufweist.

11. Verfahren zum Erzeugen einer transgenen Pflanze mit nicht dehiszenten Antheren und/oder veränderter Fruchtreife, wobei das Verfahren den Schritt des Transformierens einer Pflanzenzelle mit einem Nukleinsäurekonstrukt umfassend eine ein *ps-2-*Allel kodierende Nukleotidsequenz umfasst, wobei das *ps*-2-Allel ein Allel ist, das als letztes Nukleotid des 3'-Endes des fünften Exons des Dehiszenz-Polygalacturonasegens ein C, A oder T aufweist, wobei die Dehiszenz-Polygalacturonase ein Polypeptid mit Dehiszenz-Polygalacturonaseaktivität ist, wobei das Polypeptid eine Aminosäuresequenz, die mindestens 90% Sequenzidentität mit der Aminosäuresequenz von SEQ ID NO: 2 aufweist, oder ein Komplement hiervon umfasst, wobei die Gegenwart des Nukleinsäurekonstrukts in einer Zelle der Pflanze die Expression der Dehiszenz-Polygalacturonaseaktivität auf ein Niveau verringert, das positionelle Sterilität und nicht dehiszente Antheren und/oder veränderte Fruchtreife bewirkt.

12. Verfahren nach Anspruch 11, wobei das Nukleinsäurekonstrukt ein Konstrukt für die homologe Rekombination ist, und wobei die Nukleotidsequenz in dem letzten Nukleotid des fünften Exons des Dehiszenz-Polygalacturonasegens ein C, A oder T aufweist, das die Expression der Dehiszenz-Polygalacturonaseaktivität auf ein Niveau verringert, das positionelle Sterilität und nicht dehiszente Antheren und/oder veränderte Fruchtreife bewirkt.

13. Verfahren zum Nachweisen einer Pflanze mit einer Mutation in einem Dehiszenz-Polygalacturonase-Allel, wobei das Verfahren das Überprüfen von Pflanzen auf das Vorhandensein eines C, A oder T im letzten Nukleotid des fünften Exons des Dehiszenz-Polygalacturonasegens umfasst, wobei die Dehiszenz-Polygalacturonase ein Polypeptid mit Dehiszenz-Polygalacturonaseaktivität ist, wobei das Polypeptid eine Aminosäuresequenz umfasst, die mindestens 90% Sequenzidentität mit der Aminosäuresequenz von SEQ ID NO: 2 aufweist.

## Revendications

1. Molécule d'acide nucléique comprenant une séquence de nucléotide qui code un ps-2-allèle, dans lequel le ps-2-allèle est un allèle qui présente C, A ou T en tant que dernier nucléotide de l'extrémité 3' du cinquième exon du gène de la polygalacturonase de déhiscence, dans lequel la polygalacturonase de déhiscence est un polypeptide qui présente une activité de polygalacturonase de déhiscence, dans lequel le polypeptide comprend une séquence d'acides aminés qui présente 90 % au moins d'identité de séquence avec la séquence d'acides aminés de la séquence ID N° 2, ou une molécule d'acide nucléique qui comprend une séquence de nucléotides de 10 nucléotides contigus au moins à partir de la séquence ID N° 1 et qui comprend C, A ou T à la position 3772 de la séquence ID N° 1.

2. Molécule d'acide nucléique selon la revendication 1, dans lequel le polypeptide qui présente une activité de polygalacturonase de déhiscence peut être obtenu à partir d'espèces faisant partie du genre *Solanum.*

3. Molécule d'acide nucléique selon la revendication 2, dans lequel le polypeptide qui présente une activité de polygalacturonase de déhiscence peut être obtenu à partir d'espèces faisant partie du complexe *Solanum Lycopersicum* ou à partir du *Solanum Melongena.*

4. Procédé destiné à détecter, isoler, amplifier et / ou analyser un allèle de polygalacturonase de déhiscence dans une plante, le procédé comprenant une étape consistant à fournir un échantillon qui comprend des acides nucléiques de la plante et à hybrider les acides nucléiques de la plante avec une molécule d'acide nucléique qui comprend une séquence de nucléotide de 10 nucléotides contigus au moins d'une séquence de nucléotide selon la revendication 1.

5. Procédé selon la revendication 4, dans lequel l'allèle de polygalacturonase de déhiscence est un ps-2-allèle, dans lequel le ps-2-allèle est un allèle qui présente C, A ou T en tant que dernier nucléotide de l'extrémité 3' du cinquième exon du gène de la polygalacturonase de déhiscence, de préférence l'allèle présente C en tant que dernier nucléotide de l'extrémité 3' du cinquième exon du gène de la polygalacturonase de déhiscence.

6. Utilisation d'une molécule d'acide nucléique qui comprend une séquence de nucléotide de 10 nucléotides contigus au moins à partir d'une séquence de nucléotide selon l'une quelconque des revendications 1 à 3 dans le cadre d'un élevage assistée par marqueur.

7. Utilisation selon la revendication 6, dans laquelle la molécule d'acide nucléique est utilisée dans le cadre d'un élevage assistée par marqueur de façon à produire des plantes qui présentent un phénotype stérile positionnel dû à des anthères non déhiscentes et / ou à modifier la maturation d'un fruit.

8. Procédé destiné à produire une plante avec une mutation dans un allèle de la polygalacturonase de déhiscence, dans lequel le procédé comprend les étapes consistant à :
a) provoquer une mutation de graines d'un complexe de plante ;
b) faire pousser les plantes des graines qui ont subi une mutation obtenues en a) ; et
c) trier les plantes obtenues en b) pour détecter la présence de C, A ou T dans le dernier nucléotide du cinquième exon du gène de la polygalacturonase de déhiscence, dans lequel la polygalacturonase de déhiscence est un polypeptide qui présente une activité de polygalacturonase de déhiscence, dans lequel le polypeptide comprend une séquence d'acides aminés qui présente 90 % au moins d'identité de séquence avec la séquence d'acides aminés de la séquence ID N° 2.

9. Procédé selon la revendication 8, dans lequel le procédé est un procédé destiné à produire une plante avec un phénotype stérile positionnel dû à des anthères non déhiscentes et / ou une plante avec une maturation modifiée.

10. Procédé selon la revendication 9, dans lequel la mutation dans l'allèle de la polygalacturonase de déhiscence fait que l'allèle présente C en tant que dernier nucléotide de l'extrémité 3' du cinquième exon du gène de la polygalacturonase de déhiscence.

11. Procédé destiné à produire une plante transgénique qui présente des anthères non déhiscentes et / ou une maturation de fruit modifiée, dans lequel le procédé comprend une étape consistant à transformer une cellule de plante avec une construction d'acide nucléique qui comprend une séquence de nucléotide qui code un ps-2-allèle, dans lequel le ps-2-allèle est un allèle qui présente C, A ou T en tant que dernier nucléotide de l'extrémité 3' du cinquième exon du gène de la polygalacturonase de déhiscence, dans lequel la polygalacturonase de déhiscence est un polypeptide qui présente une activité de polygalacturonase de déhiscence, dans lequel le polypeptide comprend une séquence d'acides aminés qui présente 90 % au moins d'identité de séquence avec la séquence d'acides aminés de la séquence ID N° 2, ou un complément de celui-ci, dans lequel la présence de la construction d'acide nucléique dans une cellule de la plante réduit l'expression de l'activité de la polygalacturonase de déhiscence à un niveau qui affecte la stérilité positionnelle et les anthères non déhiscentes et / ou la maturation de fruit modifiée.

12. Procédé selon la revendication 11, dans lequel la construction d'acide nucléique est une construction de recombinaison homologue et dans lequel la séquence de nucléotide comprend C, A ou T dans le dernier nucléotide du cinquième exon du gène de la polygalacturonase de déhiscence qui réduit l'expression de l'activité de la polygalacturonase de déhiscence à un niveau qui affecte la stérilité positionnelle et les anthères non déhiscentes et / ou la maturation de fruit modifiée.

13. Procédé destiné à détecter une plante qui présente une mutation dans un allèle de la polygalacturonase de déhiscence, dans lequel le procédé comprend une étape consistant à trier des plantes pour détecter la présence de C, A ou T dans le dernier nucléotide du cinquième exon du gène de la polygalacturonase de déhiscence, dans lequel la polygalacturonase de déhiscence est un polypeptide qui présente une activité de polygalacturonase de déhiscence, dans lequel le polypeptide comprend une séquence d'acides aminés qui présente 90 % au moins d'identité de séquence avec la séquence d'acides aminés de la séquence ID N° 2.


### SEQUENCE LISTING

<110> Western Seed International B.V.
<120> Novel polygalacturonases and their uses
<130> P6015073PCT
<150> EP 07113263.3
   <151> 2007-07-26
<150> US 60/952,046
   <151> 2007-07-26
<160> 2
<170> PatentIn version 3.3
<210> 1
   <211> 6877
   <212> DNA
   <213> Solanium lycopersicum
<220>
   <221> misc_feature
   <222> (4386)..(4386)
   <223> n is a, c, g, or t
<400> 1
   aataattagt agattccaaa gatataataa tatggagaaa ttcaatgaag aagaagatca      60
   agctaaggtt acaacaatta atgtggatag ctttggagct aaaggtgatg gaagtataga     120
   tgatacaaat gtaagtatta gttttctttt aaaaaaaaat tctctttcga actattatta     180
   aaaacacgtg aatttcttat gaaaatttct cagacaaatc atcgtatatt gacttgttaa     240
   aagcttttca gacagacacg cttccattca aaaaaaataa ttaaaattca catgttttta     300
   atagtgaaac aatatatgta tactaccttg atttgattta atttatttgg cgatatttga     360
   tttaagttaa aattaaaaat aaatgaagat tttaaaactt atgatgttaa ttagatgtcc     420
   tataattttt caatggttgt aaaggcttaa atgtatatat aaaaatagga tgtatataaa     480
   gttacattat tttaaaatat aaataaaata tttttattag tcatacatac aaaatgaaat     540
   aaaagaagta tataaagtta cattatttta aaatataaat aaaatatttt tattagtcat     600
   acatataaaa tgaaataaaa gaagtatatt attagatata aaagtttgtg aagaaatgct     660
   ttttttttta aaaagaattt aatttgtatg caacgataag ttgataactg ataacatatg     720
   tcacatcttt gcgggttgat ttgcattagt tattggtgtt aaaatcaaat aaatcaattt     780
   atccggtctt ataattatta aattaaagta aatctaaaat aatatatatt tatcgatttg     840
   attgttattg gtttcagttt gattagatta tttgattact aatcatatat aaaattaaaa     900
   gaaataattt attaaaaacg tgaaaagagc ggtaacaatt tattcaaaat gaaaaatagt     960
   tagaatgact aaaattaaat aattttcaat tacagaattt actatgaata aagcttcaaa 1020
   atttactatt ttgacgtaga agaaagagac aataacattt tttgtcttaa aaaaatgatc 1080
   ataacggtta tatacatgaa accaatataa gtcaaatgtt ctcaccttga ggattattgt 1140
   ttttgcgtaa gtacaaatca taaattttaa tgaaaacaaa tataatatct ttaaaaatcg 1200
   tctacaaaaa taatatatta tatatgtatg ataaaatata atatatgtat atgatttatc 1260
   gatttgattc agttatttct ttgatatttt tctagcaaaa tcacaatcaa actaaatact 1320
   attgattttt aaaaatccaa aatcaaatca aaccataatc caaattcaat ttgtatcaat 1380
   tttatccaaa cgtgaacact cctagtcaca taacttaaaa attaattata agtaaccatc 1440
   aataagaaat gaccgaacta gttaattaat ataagtatac acacacatta attaattaaa 1500
   catctttttt cattcgatat tgatattgaa gctcaattgt ttcgactttg tcctgcgtaa 1560
   ggcctcattt ggcaaaaaaa tactctttag caagaatttt ttttatatgc aaaactcaaa 1620
   tctgagactc cgaattaaga taggagaatt aaacatctca tttcttggtg gaaaataaag 1680
   attatgtcat acttagaagc caattattat catggtgaat atatgtagca ggttgggatt 1740
   acttctacga cagaatttta tcagcatcat aagcaattta attagttttt tttaataaat 1800
   taattaatat attaaaataa atttatctta atttcaggca tttcaaaaag catggaaaga 1860
   agcttgttca tcttcacatg ttgtgaattt tgtggtgtcc cagaacaaga aatatcttct 1920
   caaaccaatc aaattttatg ggccatgcaa atcttccatt acaatgcagg ttaggttgac 1980
   atatcattat gtaaaaaaaa aactcgaagt aacgataaaa ttattctcgt cctaatcact 2040
   ataaagtatg gatgttctga gtcatggtca agtaattaga tcttgaggct tgcatcaggg 2100
   taggctgttt gtgtcatggt gtggctcttt ccaaaactta tatgaaagtg agatgctttg 2160
   tgcatccgat tatcgattca aactaacaga ataaaagttt tttttatacg attaggttat 2220
   tttaaaatat gataaataat cttattcgac gagttgaatt gcatcgtgta tgtggatcta 2280
   tagtgtaatc taaacattta cgcgacttta ttgaatagta tccaaaaaat attaaatcag 2340
   aaattgatat tgttatataa acggataagt ttcaaatctt gaatacacct ttgataatat 2400
   gaaaaactta ttacattgtc tgcgcacgga tataagttaa ttaaactctg ctattattat 2460
   atatatgtag agaaataagc tctatgtata gtgacaaagc tatatccgtt gaacgatgat 2520
   tagttgaaca cttaggttaa aatataacct taattttata tattgcgaca cctttcgtga 2580
   aatttttaaa tctcgtcacc acttttagtt acatttggaa ataaataata ggttggagtg 2640
   tagaatatta ttttgacact tttgttcaat ttttttttat tgtagattta tggaacccta 2700
   ttagcatctg atgatacttc agattacaag aaggatagta ggcactggct tatttttgat 2760
   agtgttcaaa aattggttgt tggaggagct ggagttatca atggcaatgg caaaatttgg 2820
   tggcaacatt cttgcaaaat taataaaaaa ttggtataaa tacatactta attttgaaag 2880
   ggtcgagaat cttgaaacaa tcctaaagtt tataggtcac cgtcagttcg aatcgtgtgc 2940
   aaataattac tattatttgc actaggatag actgtctaca ttatacgcca atcaagtgca 3000
   accttcccaa accttgcata agtgcatgat gttttatgag ccggggccgg aaagcccttt 3060
   tgtcctttac atacttaatt ttaaaaggag agtcttggag taatgataaa attgtctatg 3120
   tgcatgtgat tataggttac tgtccgttca atccgtgtag gaacaattac taatacatgt 3180
   tttagaatag actatctata ttacactcca atcaagtgca gttctttccg aaccttgcat 3240
   aaacacatga tgttttatgc aacggggcgt gggcaaccct tttgcccttt gcaaacttta 3300
   tttatttatt tatatatata ttcctatttg tttcgtttgt ttttatgata tataactaat 3360
   tttgtctaat ttttattcgt attgtctact ttttttttta ttttgcagcc atgcaaggta 3420
   gcacccacgg tacgtttatt tgatttgatt tcttattttt caaaagaaca aaacaattaa 3480
   ttattacgtc atttttcttt aatatttttt ttgttgatag agatttttta ttttatttta 3540
   ttggtgacat ttaaattttt tcaggccctg acattttaca agtgtaacaa cttgaaagtg 3600
   aaggacctta aaatagaaaa tgcacaacaa atacatttgc taattgagaa gtgtgttggt 3660
   gttgaagtta caaaattggt agtgacttct ccagaaaata gccctaatac tgatggaatc 3720
   catataacta gcactcaaaa tattcaaatt tctgattcca ccattgccac aggtatcgat 3780
   aaattaacga ttttaatttt tatatactta tagtaaaaaa taattatatt atcaagttgt 3840
   atgtataaat taaaggtaaa catgctttta ataagtgaaa cttatgataa gttattagcc 3900
   tgaaaatatg aaagattaaa attttcgaca attgatcatc atcgtctcat cgaccacttg 3960
   gtctttgacg tagggctttt cctaattccc gaatgaactt gataagtcaa ctcaaagata 4020
   aatatatgtt tagatcatga gtaacgtgtg ataccttagg taaatttcac ttcgatatac 4080
   atataagaaa tgttggtgta taggaaaaca caatttagac aatagtgtca atgtggtttg 4140
   atttaaaagg tgcgattggt ataaaggaaa acatttttca gaatcaactc attttcctca 4200
   aaattaagga taatgaattc ccttcaaaaa ttgaggaaaa catttttcaa aactctcatc 4260
   caatttgaaa ttatatattt tttaaagaaa acatcgattt cgaaaaatat attcaatttc 4320
   aaaattttat atattcacca ccaccccacc ccacccgatc atgccttcac cccccccccc 4380
   ccccanaaaa aaaatagatt ttttttttaa aaaaatattt ttaatttcaa aatttcattt 4440
   ctccatccct accctttacc ccccccccct accaaccctc tacccccctc ccaaaaaaga 4500
   aaattaagtt tgtcttaaaa aatattttcg acttcaaatt tttatttttt cacccttacc 4560
   tcgacccccg ccccccaaca cccaccccac cccactccat ctttaaagat aaattttcat 4620
   tttgtttttt taaaaatatt tttaacctca aatttttatt ttttctaccc ctacgtcccc 4680
   ctctccagtc cccttaccat ccctccagcc ccaccaaaaa aatatacttt aatttttaaa 4740
   aaatattttc aactttaaat ttttattttt tcactcctaa cctcccgcac ccacccaccc 4800
   ccgccagccc cccacccccc aaaaaaaatt attttcaatt tcaaaaatta gtttgtactc 4860
   tagtaaaaat aaaagatatt tctcaaaaat atttttcatt cataaattaa acactaaaaa 4920
   atattttcta ctcatcaacc aaacatgaaa aaacaaatca taaatctact tattttctaa 4980
   aaaaaaaaca ttttcctaat taccaaacac accgaaaatc ttgaaagtgt agttccaagc 5040
   ggataattaa tgtcactata gtgagttgta ctattataga ttgttgaaat aatatttaca 5100
   aatattaaat tatactaatt gtacccaaaa aaaatgacat taaatttttt tatttactaa 5160
   ctatgtattt tggaaataat ttttgtggta ggtgatgatt gcatctcaat tgtggatgga 5220
   tctcagaagg tcttagccac tggcattact tgtggaccag gtcatggaat taggtaaaaa 5280
   caaaataatt tcattatatt agtattatta tgctagtatt atcttatttt ttattatatt 5340
   attagtgtta tttctctaat ttttattatc tttactttca ttacttttct ttctttaata 5400
   tctttatcat tttatttttg tatatctttt gaactgttct gaaaaaattg tttttttatc 5460
   gtttatatga aacaatctcc taccccacca aggtaagaaa taagtttttg ggtcaaacct 5520
   cacttgcatg attattttct ttatcatatt cagattattt tatcaaaatt tataatttaa 5580
   ttatgtaatt tttttttatt attattattt gtgcttagta ttggaagttt gggaggtgga 5640
   aattcagaag ctcatgtgtc tgatattcat gtaaatggag ctaagcttta tgaaactaca 5700
   aatggactta ggattaagac ttggccggta aattaattaa ttaattattt ttattaattt 5760
   acaaatattc tttataatta atatacatat aattatattc ttgaattatt ttgtataggg 5820
   aggatttgga agtgcaagca atattaagta tcaaaatgtg gttatgaata atgtcaaaaa 5880
   tccaataatt atagaccaaa attattgtga tcaagctgat ggtccatgca aagctgaggt 5940
   aattgaaaat gaatttaact tatatatatt aactatacga aaaaataatc tactttgtta 6000
   aagcagatat cttatcgtat tttttagata attaatccgt tccattatgg gttgttagtt 6060
   aagctatctt tagatagcca aatatggtaa attttttaat aatgtcgaca tatgtatgtt 6120
   ataaaaatat ataacttaaa ctcgaacaaa aatgaatttt atttatataa catacttaat 6180
   gtatagaata tttttatacc attagtgcaa tttgctacat catttggaca atatagtgtt 6240
   ttaacttata aacaaaaata atttctcccc ttcaactttc ttaaaattca ataaaatccc 6300
   catacaaaat attgtataat caaatttacc ccactttgaa aatgtaacat ataacgacat 6360
   attgtacatt ataaagttgt gacggaatat ctatgacgat aatattaatg acagagttaa 6420
   tatggtcgat tcataatatg attcaatcac aaacttgtga taatttcatt gatctcttaa    6480
   tcacaaaaat atatttatac atttatagac tgactcggca gttgaagtga aaaatgtgat    6540
   ttatcaaaat atcaaaggca caagtgcaac aaatgatgca ataagtatca agtgcagcaa    6600
   aaaaattcca tgtgaaggaa ttttgatgga gaatgtgaaa ttgttaggag gaaatggtga    6660
   aactccaaat ggtatttggg gaaatatcaa taatcttacg tgcaaaaatg ttttaccaga    6720
   atgtcaaaaa aactcaaaaa ttgtataatt aagtttgata gagttaatta atgtttaata    6780
   ggatattgta tagttataca tatgacaaat taagtgttag aatacataat tagcaaatga    6840
   tttgtgataa atgatattct ttgtacacat gaaataa                             6877
<210> 2
   <211> 319
   <212> PRT
   <213> Solanium lycopersicum
<400> 2
   Cys Ser Ser Ser His Val Val Asn Phe Val Val Ser Gln Asn Lys Lys
   1               5                   10                  15
   Tyr Leu Leu Lys Pro Ile Lys Phe Tyr Gly Pro Cys Lys Ser Ser Ile
               20                  25                  30
   Thr Met Gln Ile Tyr Gly Thr Leu Leu Ala Ser Asp Asp Thr Ser Asp
           35                  40                  45
   Tyr Lys Lys Asp Ser Arg His Trp Leu Ile Phe Asp Ser Val Gln Lys
       50                  55                  60
   Leu Val Val Gly Gly Ala Gly Val Ile Asn Gly Asn Gly Lys Ile Trp
   65                  70                  75                  80
   Trp Gln His Ser Cys Lys Ile Asn Lys Lys Leu Pro Cys Lys Val Ala
                   85                  90                  95
   Pro Thr Ala Leu Thr Phe Tyr Lys Cys Asn Asn Leu Lys Val Lys Asp
               100                 105                 110
   Leu Lys Ile Glu Asn Ala Gln Gln Ile His Leu Leu Ile Glu Lys Cys
           115                 120                 125
   Val Gly Val Glu Val Thr Lys Leu Val Val Thr Ser Pro Glu Asn Ser
       130                 135                 140
   Pro Asn Thr Asp Gly Ile His Ile Thr Ser Thr Gln Asn Ile Gln Ile
   145                 150                 155                 160
   Ser Asp Ser Thr Ile Ala Thr Gly Asp Asp Cys Ile Ser Ile Val Asp
                   165                 170                 175
   Gly Ser Gln Lys Val Leu Ala Thr Gly Ile Thr Cys Gly Pro Gly His
               180                 185                 190
   Gly Ile Ser Ile Gly Ser Leu Gly Gly Gly Asn Ser Glu Ala His Val
           195                 200                 205
   Ser Asp Ile His Val Asn Gly Ala Lys Leu Tyr Glu Thr Thr Asn Gly
       210                 215                 220
   Leu Arg Ile Lys Thr Trp Pro Gly Gly Phe Gly Ser Ala Ser Asn Ile
   225                 230                 235                 240
   Lys Tyr Gln Asn Val Val Met Asn Asn Val Lys Asn Pro Ile Ile Ile
                   245                 250                 255
   Asp Gln Asn Tyr Cys Asp Gln Ala Asp Gly Pro Cys Lys Ala Glu Thr
               260                 265                 270
   Asp Ser Ala Val Glu Val Lys Asn Val Ile Tyr Gln Asn Ile Lys Gly
           275                 280                 285
   Thr Ser Ala Thr Asn Asp Ala Ile Ser Ile Lys Cys Ser Lys Lys Ile
       290                 295                 300
   Pro Cys Glu Gly Ile Leu Met Glu Asn Val Lys Leu Leu Gly Gly
   305                 310                 315